# EUROPEAN PATENT SPECIFICATION

(11) **EP 1 315 816 B1**
(45) Date of publication and mention of the grant of the patent: **29.12.2010**
(21) Application number: 01972985.4
(22) Date of filing: 10.09.2001
(51) Int. Cl.: C12N 15/40, C12N 15/00, A61K 39/21

(54) **GENETICALLY ENGINEERED CO-EXPRESSION DNA VACCINES, CONSTRUCTION METHODS AND USES THEREOF**
GENMANIPULIERTE DNA-VAKZINE ZUR CO-EXPRESSION, METHODEN ZU DEREN HERSTELLUNG UND DEREN VERWENDUNGEN
VACCINS A BASE D'ADN A CO-EXPRESSION CON US PAR GENIE GENETIQUE, LEURS PROCEDES DE REALISATION ET LEURS UTILISATIONS

(30) Priority: 08.09.2000 US 231070 P; 08.09.2000 US 231376 P; 08.09.2000 US 231403 P; 08.09.2000 US 231449 P
(43) Date of publication of application: 04.06.2003
(60) Divisional application: 10007008.5
(73) Proprietor: UNIVERSITY OF MARYLAND BIOTECHNOLOGY INSTITUTE, Baltimore, MD 21202 (US)
(72) Inventor: HONE, David, Ellicott City, MD 21043 (US); LEWIS, George, Baltimore, MD 21218 (US); FOUTS, Timothy, Columbia, MD 21045 (US); BAGLEY, Ken, Baltimore, MD 21234 (US); BOYSON, Michael, Seattle, Washington 98115 (US); OBRIECHT, Christine, Ellicott City, MD 21041 (US); SHATA, MT, Pine Bush, NY 12566-6729 (US); AGWALE, Simon, Randallstown, MD 21133 (US)
(74) Representative: Schüssler, Andrea
(86) International application number: PCT/US2001/028365
(87) International publication number: WO 2002/019968

(56) References cited:
- EP-A1- 0 803 573
- REKOSH D ET AL.: "Coexpression of human immunodeficiency virus envelope proteins and tat from a single simian virus 40 late replacement vector" PROC. NATL. ACAD. SCI. USA, vol. 85, January 1988 (1988-01), pages 334-338, XP002334350
- HINKULA J ET AL: "Nucleic acid vaccination with HIV regulatory genes: a combination of HIV-1 genes in separate plasmids induces strong immune responses" VACCINE, BUTTERWORTH SCIENTIFIC. GUILDFORD, GB, vol. 15, no. 8, June 1997 (1997-06), pages 874-878, XP004075674 ISSN: 0264-410X
- BOYER J D ET AL: "THERAPEUTIC IMMUNIZATION OF HIV-INFECTED CHIMPANZEES USING HIV-1 PLASMID ANTIGENS AND INTERLEUKIN-12 EXPRESSING PLASMIDS" AIDS, LONDON, GB, vol. 14, no. 11, 28 July 2000 (2000-07-28), pages 1515-1522, XP009049682 ISSN: 0269-9370
- KIM J J ET AL: "CHEMOKINE GENE ADJUVANTS CAN MODULATE IMMUNE RESPONSES INDUCED BY DNA VACCINES" JOURNAL OF INTERFERON AND CYTOKINE RESEARCH, MARY ANN LIEBERT, NEW YORK, NY, US, vol. 20, May 2000 (2000-05), pages 487-498, XP002908473 ISSN: 1079-9907
- SHIVER J W ET AL: "Anti-HIV env immunities elicited by nucleic acid vaccines" VACCINE, BUTTERWORTH SCIENTIFIC. GUILDFORD, GB, vol. 15, no. 8, June 1997 (1997-06), pages 884-887, XP004075676 ISSN: 0264-410X
- YANJUN LIU, HAO WANG, JIAN ZHAO ET AL: "Enhancement of immunogenicity of tumor cells by cotransfection with genes encoding antisense insulin-like growth factor-1 and B7.1 molecules" CANCER GENE THERAPY, vol. 7, no. 3, March 2000 (2000-03), pages 456-465, XP002369384
- BOYER JEAN D ET AL: "Protection of chimpanzees from high-dose heterologous HIV-1 challenge by DNA vaccination" NATURE MEDICINE, NATURE PUBLISHING, CO, US, vol. 3, no. 5, 1997, pages 526-532, XP002165137 ISSN: 1078-8956
- ETCHART N.: 'Class-1 -restricted CTL induction by mucosal immunization with naked DNA encoding measles virus haemagglutinin' JOURNAL OF GENERAL VIROLOGY vol. 78, 1997, pages 1577 - 1580, XP002908472
- PRAYAGA S.K. ET AL.: 'Manipulation of HIV-1 gp120-specific immune response elicited via gene gun-based DNA immunization' VACCINE vol. 15, no. 12/13, 1997, pages 1349 - 1352, XP002097491
- LEITER W.W. ET AL.: 'DNA and RNA -based vaccines: principles, progress and prospects' VACCINE vol. 18, 2000, pages 765 - 777, XP002942637
- KUKLIN N. ET AL.: 'Induction of mucosal immunity against herpes simplex virus by plasmid DNA immunization' JOURNAL OF VIROLOGY vol. 71, no. 4, April 1997, pages 3138 - 3145, XP002908471
- CASELLI E. ET AL.: 'DNA immunization with HIV-1 tat mutated in the trans activation domain induces humoral and cellular immune responses against wild-type tat1' THE JOURNAL OF IMMUNOLOGY vol. 162, 1999, pages 5631 - 5638, XP002129597
- GAN W. ET AL.: 'Functional characterization of the internal ribosome entry site of eIF4G mRNA' THE JOURNAL OF BIOLOGICAL CHEMISTRY vol. 273, no. 9, 27 February 1998, pages 5006 - 5012, XP002908478
- BECKER Y.: 'HIV-peplotion vaccine, a novel approach to vaccination against ADIS by transepithelial transport of viral peptides and antigens to langerhans cell for induction of cytolytic T cells by HLA class I and CDI moleuclar for long term protection' COHEN AND SHAFFERMAN 1996, pages 97 - 104, XP002908477
- LEE J.L. ET AL.: 'Induction of an antigen-specific, CD1-restricted cytotoxic T lymphocyte response in vivo' JOURNAL EXP. MED. vol. 187, no. 3, 1998, pages 433 - 438, XP002908476
- BOYER J.D. ET AL.: 'HIV-1 DNA vaccines and chemokines' VACCINE vol. 17, 1999, pages S53 - S64, XP002908475
- MACGREGOR R.R. ET AL.: 'First human trial of a DNA-based vaccine for treatment of HIV-1 infection: safety and host response' J. OF INFECTIOUS DISEASES vol. 178, 1998, pages 92 - 100, XP000982247
- MELLSTEDT ET AL.: 'Augmentation of the immune response with granulocyte-macrophage colony-stimulating factor and other hematopoietic growth factors' CURRENT OPINION IN HEMATOLOGY vol. 6, 1999, pages 169 - 175, XP002908474
- SASAKI S. ET AL.: 'Comparison of intranasal immunization against human immunodeficiency virus type 1 with a DNA-monophosphoryl lipid A adjuvant vaccine' INFECTION AND IMMUNITY vol. 66, no. 2, 1998, pages 823 - 826, XP002128173
- KIM J.J. ET AL.: 'Chemokine gene adjuvants can modulate immune response induced by DNA vaccines' JOURNAL OF INTERFERON AND CYTOKINE RESEARCH vol. 20, 2000, pages 487 - 498, XP002908473
- WILD ET AL.: "Polyvalent vaccination against hepatitis B and core antigen using a dicistronic expression plasmid." VACCINE, vol. 16, no. 4, 1998, pages 353-360, XP004099294

## Description

### FIELD OF THE INVENTION

The present invention provides co-expression DNA-vaccines and methods of increasing an immune response to HIV in a subject. In particular, the present invention relates to dicistronic DNA vaccines including a plasmid comprising an HIV-antigen-encoding region encoding an antigen component, wherein the antigen component is gp120 or hgp120, in combination with a biologically-active component-encoding region encoding an immunregulatory peptide, wherein the immunoregulatory peptide is the A1 domain of the A subunit of cholera toxin or the A subunit of *E. coli* heat labile enterotoxin. Further, the present invention relates to a single expression cassette including a nucleotide sequence comprising the HIV antigen-encoding region and a biologically-active component-encoding region. Still further, the present invention provides an expression vector for use in increasing an immune response to HIV in a subject, wherein the expression vector comprises the HIV antigen-encoding region and a biologically-active component-encoding region which retains some or all of the biological activity of the native domain.

### BACKGROUND OF THE INVENTION

Conventional DNA vaccines axe normally produced as a plasmid that can be introduced into animal tissue and therein expressed by animal cells to produce a messenger ribonucleic acid (mRNA) molecule, which is translated to produce one protein, one fragment of a protein or one fusion protein.

A diverse array of conventional DNA vaccines are known in the art. These vaccines generally include a plasmid vector, a promoter for transcription initiation that is active in eukaryotic cells, and a vaccine antigen (Gurunathan, et al., Ann. Rev. Immunol., 18:927 (2000); Krieg, Biochim, Biophys. Acta., 1489:107 (1999); Cichutek, Dev. Biol. Stand., 100:119 (1999); Davis, Microbes Infect, 1:7 (1999); Leitner, Vaccine, 18:765 (1999)).

Examples of plasmid vectors that have been used in conventional DNA vaccines include pBR322 (ATCC# 31344); pUC19 (ATCC# 37254); pcDNA3.1 (Invitrogen, Carlsbad CA 92008; Cat. NO. V385-20; DNA sequence available at www.invitrogen.com/vectordata/index.html); *pNGVL (National Gene Vector Laboratory, University of Michigan, MI); p414cyc (ATCC# 87380), p414GALS (ATCC# 87344), pBAD18 (ATCC# 87393), pBLCAT5 (ATCC# 77412), pBluescriptIIKS, (ATCC# 87047), pBSL130 (ATCC# 87145), pCM182 (ATCC# 87656), pCMVtkLUC (ATCC# 87633), pECV25 (ATCC#77187), pGEM-7zf (ATCC# 87048), pGEX-KN (ATCC# 77332), pJC20 (ATCC# 87113, pUB110 (ATCC# 37015), pUB18 (ATCC# 37253).

Examples of promoters that have been used in conventional DNA vaccines include the SV40 early promoter (GenBank accession # M99358, Fiers, et al., Nature, 273: 113-120 (1978)); the cytomegalovirus immediate early promoter/enhancer (GenBank accession # AF025843); and the rous sarcoma virus long terminal repeat prompter (Genbank accession # M83237; Lon, et al., Hum. Immunol., 31: 229-235 (1991)); eulcaryotic promoters or parts thereof, such as β-casein (GenBank accession # AF194986; Fan, et al., Direct submission (2000)); uteroglobin (GenBank accession # NM003357; Hay, et al., Am. J. Physiol., 268: 565-575 (1995)); β-actin (GenBank accession # NM001101; ref Vandekerckhove and Weber, Proc. Natl. Acad. Sci. U.S.A., 73: 1106-1110 (1978)); ubiquitin (GenBank accession # AJ243268; Robinson. Direct Submission, (2000)) or tyrosinase (GenBank accession # NM000372; Shibaharo, et al., Tohoku J. Exp. Med., 156: 403-414 (1988)) promoters.

Examples of vaccine antigens that have been used in conventional DNA vaccines include *Plasmodium vivax and Plasmodium falciparum antigens; Entamoeba histolytica* antigens; Hepatitis C virus antigens, Hepatitis B virus antigens, HIV-1 antigens, Semliki Forest virus antigens, Herpes Simplex viral antigens, Pox virus antigens, Influenza virus antigens, Measles virus antigens, *Dengue* virus antigens and Papilloma virus antigens. A comprehensive reference database of DNA vaccine citations is available at (www.DNAvaccine.com/Biblio/articles.htm).

Since their inception in 1993, conventional DNA vaccines encoding an antigen under the control of an eukaryotic or viral promoter have been used to immunize rodents (e.g., mice, rats and guinea pigs, swine, chickens, ferrets, non-human primates and adult volunteers (Webster, et al., Vacc., 12:1495-1498 (1994); Bernstein, et al., Vaccine, 17:1964 (1999); Huang, et al.,Virol Immunol., 12:1 (1999); Tsukamoto, et al., Virology, 257:352 (1999); Sakaguchi, et al., Vaccine, 14:747 (1996); Kodihalli, et al., J. Virol., 71: 3391 (1997); Donnelly, et al., Vaccine, 15:865 (1997); Fuller, et al., Vaccine, 15:924 (1997); Fuller, et al., Immunol. Cell Biol., 75: 389 (1997); Le, et al., Vaccine, 18:1893 (2000); Boyer, et al., J. Infect. Dis., 181:476 (2000));.

Although conventional DNA vaccines induce immune responses against a diverse array of antigens, the magnitudes of the immune responses have not always been sufficient to engender protective immunity. Several approaches have been developed to increase the immunogenicity of conventional DNA vaccines, including the use of altered DNA sequences, such as the use of antigen-encoding DNA sequences optimized for expression in mammalian cells (Andre, J. Virol., 72:1497 (1998); Haas, et al., Curr. Biol. 6:315-24 (1996); zur Megede, ., J. Virol., 74:2628 (2000); Vinner, et al., Vaccine, 17:2166 (1999); Krieg, Biochim. Biophys. Acta., 1489:107 (1999); McAdam, et al., J. Virol., 74: 203-208 (2000); Davis, Curr. Top. Microbiol. Immunol., 247:17 (2000); McCluskie, Crit. Rev. Immunol., 19:303 (1999); Davis, Curr. Opin. Biotechnol., 8:635 (1997); Lobell, J. Immunol., 163:4754 (1999)). The immunogenicity of conventional DNA vaccines can also be modified by formulating the conventional DNA vaccine in an adjuvant, such as aluminum phosphate or aluminum hydroxyphosphate (Ulmer, et al., Vaccine, 18:18 (2000)), monophosphoryl-lipid A (also referred to as MPL or MPLA; Schneerson, et al., J. Immunol., 147: 2136-2140 (1991); Sasaki, et al., Inf. Immunol., 65: 3520-3528 (1997); Lodmell, et al., Vaccine, 18: 1059-1066 (2000)), QS-21 saponin (Sasaki, et al., J. Virol., 72:4931 (1998); dexamethasone (Malone, et al., J. Biol. Chem., 269:29903 (1994); CpG DNA sequences (Davis, et al., J. Immunol., 15:870 (1998); lipopolysaccharide (LPS) antagonist (Shata and Hone, PCT International Application No. PCT/US00/27402), a cytokine (Hayashi, et al., Vaccine, 18: 3097-3105 (2000); Sin, et al., J. Immunol., 162: 2912-2921 (1999); Gabaglia, et al., J. Immunol., 162: 753-760 (1999); Kim, et al., Eur. J. Immunol., 28:1089 (1998); Barouch, et al., J. Immunol., 161:1875 (1998); Okada, et al., J. Immunol., 159:3638 (1997); Kim, et al., J. Virol., 74:3427 (2000)), or a chemokine (Boyer, et al., Vaccine, 17(Suppl 2):553 (1999); Xin, et al., Clin. Immunol., 92:90 (1999)).

Cholera toxin (Ctx) is a well-known adjuvant that is typically used to augment the immunogenicity of mucosal vaccines, such as those given intranasally or orally (Xu-Amino, et al., J. Exp. Med., 178:1309 (1993); VanCott, et al., Vaccine, 14:392 (1996); Jackson, R. J. et al., Infect. Immun., 61:4272 (1993); Marinaro, M. et al., Ann. New York Acad. Sci., 795:361 (1996); Yamamoto, S. et al., J. Exp. Med., 185:1203 (1997); Porgador, et al., J. Immunol., 158:834 (1997); Lycke and Hohngren, Monogr., Allergy, 24:274 (1988); Hornquist and Lycke, Eur. J. Immunol., 23:2136 (1993); Homquist, et al., Immunol., 87:220 (1996); Agren, et al., Immunol. Cell Biol., 76:280 (1998)). The adjuvant activity of Ctx is mediated by the A1 domain of the A subunit of Ctx (herein referred to as CtxA1); chimeric proteins comprised of an antigenic protein fused to the CtxA1 protein demonstrate that CtxA1 alone possesses adjuvant activity (Agren, et al., J. Immunol., 164:6276 (2000); Agren, et al., Immunol. Cell Biol., 76:280 (1998); Agren, et al., J. Immunol., 158:3936 (1997)). The utilization of the A subunit, the A1 domain of Ctx or analogues thereof in a DNA vaccine has not heretofore been reported.

More recently the use of Ctx as an adjuvant has been extended to transcutaneous vaccines (Glenn, et al., Infect. Immun., 67:1100 (1999); Scharton-Kersten, et al., Vaccine ,17(Suppl. 2):S37 (1999)). Thus, recent evidence suggests that cholera toxin (CT) as an adjuvant applied topically with an antigen to the skin surface (i.e. transcutaneous vaccination) elicits IgG responses against the antigen, whereas topical application of the antigen alone does not induce detectable IgG response (Glenn, et al., *supra* (1999); Scharton-Kersten, et al., *supra* (1999)).

Although the function of the human immunodeficiency virus (HIV) Tat protein in viral RNA expression is well established, the auxiliary functions ascribed to Tat remain controversial (Gallo, Proc. Natl. Acad. Sci. 96:8324 (1999)). A growing consensus suggests that Tat possesses immunoregulatory properties (Viscidi, et al., Science, 246:1606 (1989); Frankel, et al., Proc. Natl. Acad. Sci., 86:7397 (1989); Frankel and Pabo, Cell, 55:1189 (1988); Ito, et al., AIDS Res. Hum. Retroviruses, 14:845 (1998); Wrenger, et al., J Biol Chem., 272:30283-8 (1997); Wrenger, et al., FEBS Lett., 383:145 (1996); Wrenger, et al., J. Biol. Chem., 272:30283 (1997); Zauli, et al., Blood, 80:3036 (1992); Lachgar, et al., Biomed. Pharmacother., 50:13 (1996)). In one study, recombinant Tat was shown to inhibit antigen-specific T cell proliferation in peripheral blood mononuclear cells (PBMCs) collected from healthy volunteers (Viscidi, et al., Science, 246:1606 (1989)). In another independent study, recombinant Tat inhibited the proliferation of purified T cells in response to immobilized CD3-specific monoclonal antibodies (Meyaard, et al., Eur. J. Immunol., 22: 2729-2732 (1992)). The latter result suggests that the immunoregulatory activity of Tat is exerted directly on T cells. In agreement, Westendorp, et al., (Westendorp, et al., Nature, 375: 497-500 (1995)) showed that purified Tat sensitized a T cell line and enriched primary CD4⁺ and CD8⁺ T cells to cell death by apoptosis. Oxidation of Tat (Westendorp, et al., *supra,* (1995)) or pretreatment of Tat with anti-Tat monoclonal antibodies (McCloskey, et al., J Immunol., 158:1014 (1997)) was shown to eliminate the pro-apoptotic activity, suggesting that a native Tat structure and binding to a cellular target may be central to the apoptosis activity.

In contrast, expression of Tat by transfected Jurkat cells did not render them sensitive to anti-CD3-mediated apoptosis (McCloskey, et al., *supra* (1997); Gibellini, et al., Br. J. Haematol., 89:24 (1995)). Thus, Tat might protect HIV-infected T cells from pro-apoptotic signals and yet exert pro-apoptotic affects on uninfected T cells (McCloskey, et al., *supra* (1997); Gibellini, et al., *supra* (1995)).

Tat has been shown to alter the function of antigen presenting cells (herein referred to as APCs). Data have been reported showing that extracellular Tat induces interferon-α secretion by peripheral blood mononuclear cells, which in turn suppresses lymphocyte proliferation (Zagury, et al., Proc. Nat. Acad. Sci. U.S.A, 95: 3851-3856 (1998)). Another report suggests that the RGD domain of Tat inhibits the uptake of apoptotic blebs by dendritic cells (Zoch, et al., AIDS, 11:1227-1235 (1997)). Yet another report indicated that Tat may induce transforming growth factor-beta (TGF-β) expression, which is know to alter APCs and impart immunosuppressive activity (Gibellini, et al., Br. J. Haematol., 88:261 (1994); Zauli, et al., Blood, 80:3036 (1992)). Collectively, these observations suggest that the immunoregulatory activity of Tat may encumber a broad array of immune cell subsets.

A common prediction among previous studies, is that infected cells release Tat and that exogenous Tat subsequently imparts its immunoregulatory affects on mononuclear cells patrolling the foci of an HIV infection (Viscidi, et al., *supra* (1989); Frankel, et al., *supra* (1989); Frankel and Pabo, *supra* (1988); Ito, et al., *supra* (1998); Wrenger, et al., *supra* (1997); Wrenger, et al., *supra* (1996); Wrenger, et al., *supra* (1997); Zauli, et al., *supra* (1992); Lachgar, et al., *supra* (1996); Zocchi, et al. *supra* (1997)). In support of this notion, it is believed that soluble exogenous Tat is transported by an as yet undefined mechanism into the intracellular compartment (e.g., the cytoplasm and nucleus) of cell cultured *in vitro* (Vives, et al., J. Biol. Chem., 272:16010 (1997); Ma, et al., J. Virol., 71:2495 (1997); Valvatne, et al., AIDS Res. Hum. Retroviruses, 12:611 (1996); Chen, et al., Anal. Biochem., 227:168 (1995); Ensoli, et al., J. Virol., 67:277 (1993); Mann and Frankel, EMBO J., 10:1733 (1991); Frankel and Pabo, Cell, 55:1189 (1988)). Indeed, chimeric fusion proteins incorporating the Tat transcellular uptake sequence have become a molecular tool for introducing passenger proteins into mammalian cells (Kim, et al., J. Immunol., 159:1666 (1997); Fawell, et al., Proc. Natl. Acad. Sci., 91:664 (1994)).

However, the levels of Tat (up to 10 µg/ml) used to induce anti-proliferative and pro-apoptotic effects *in vitro,* as observed in the reports cited above, may not occur during the natural course of an HIV infection (Helland, et al., J. Virol., 65:4547 (1991)). An alternative view, therefore, is that expression of Tat by T cells down regulates HLA class I levels in the infected cell (Carroll, et al., Mol. Immunol., 35:1171-1178 (1998); Matsui, et al., J. Acquir. Immune Defic. Syndr. Hum. Retrovirol., 11(3):233 (1996); Howcroft et al., Science, 260: 1320-1322 (1993)). Others have demonstrated the down regulation of HLA class II molecules (Kanazawa, et al., Immunity, 12: 61-70 (2000); Tosi, et al., Eur. J. Immunol., 30: 19-28 (2000)). These studies adhere to the notion that immunoregulation by Tat occurs primarily in HIV-infected cells. None of these reports rule out the possibility that immunoregulatory activity of Tat may be mediated through a combination of intercellular and intracellular effects.

Hence, an animal model that mimics the immunoregulatory property of Tat would be a useful tool to define the mechanism utilized by Tat to influence immune function and for the development and assessment of preventive and therapeutic strategies (e.g., pharmaceutics and vaccines) against the auxiliary function(s) (e.g., immunoregulatory) of Tat. Unfortunately, heretofore expression of Tat in transgenic mice caused pleiotropic pathological affects (Choi, et al., J Biol Chem., 275:3693 (2000); Brady, et al., J. Virol., 69: 7622 (1995); Kundu, et al., Blood, 94: 275 (1999)), making them difficult to adapt for use in controlled immunological studies. More recently, a murine model was reported in which recombinant Tat was shown to modestly suppress the serum IgG and proliferative responses to an HIV-1 p24 vaccine (Cohen, et al., Proc. Natl. Acad. Sci. U.S.A., 96: 10842-10847 (1999)). However, the immunosuppressive activity required large quantities of purified recombinant Tat, which is difficult to produce and expensive to purchase commercially. Further, the recombinant Tat used in the above-cited study was deactivated by oxidation (Cohen, et., *supra* (1999)); thus, the murine model is highly susceptible to qualitative variations in the recombinant Tat employed and its shelf half-life. Accordingly, at present there is no satisfactory laboratory animal model that reproducibly mimics the immunoregulatory properties of the HIV-1 Tat protein.

Furthermore, Hinkula, et al., Vaccine, 15:874-878 disclose a concept of combining several genes in order to immunize against a microbial agent Therefore, HIV genes that individually have been shown to mediate immune responses against HIV proteins have been selected. These proteins were the regulating genes/proteins of HTV-1 *rev, tat* and *nef* as well as structural genes for gp160 under the control of *rev,* and the capsid p24 represented by the larger precursor gene p37.

The prior art provides for conventional DNA vaccines. The immunogenicity of conventional DNA vaccines is modified by mixing the conventional DNA vaccine with an adjuvant or a second plasmid encoding an immunoregulatory protein (e.g., a cytokine or chemokines).

However, heretofore, there has been no demonstration that DNA vaccines that co-express an antigen in combination with adjuvants, immunoregulatory agents, antisense RNAs, and catalytic RNAs are more effective than a mixture of a conventional DNA vaccine and a plasmid that encodes an immunoregulatory protein. That is, until the present invention which provides the first dicistronic DNA vaccine including a plasmid comprising an HIV antigen-encoding region and a biologically-active component-encoding region encoding an immunoregulatory agent, and that is shown hereinbelow to be more effective than the sum of its parts.

### SUMMARY OF THE INVENTION

The present invention describes the novel and unexpected finding that co-expression DNA (CED) vaccines display unprecedented immunogenic properties. An important application of CED vaccines is that they are capable of inducing significantly stronger immune responses against vaccine antigens than conventional DNA vaccines. Another important application of CED vaccines is that they are capable of inducing systemic tolerance. Further, the present invention provides the first documentation that CED vaccines are more effective than the sum of the parts.

In one aspect the present invention provides for CED vaccines (...) such as a dicistronic DNA vaccine including a plasmid comprising an HIV-antigen-encoding region, wherein the antigen component is gp120 or hgp120, in combination with a biologically-active component-encoding region encoding an immunregulatory peptide, wherein the immunregulatory peptide is the A1 domain of the A subunit of cholera toxin or the A subunit of *E. coli* heat labile enterotoxin.

In another aspect the present invention provides a single expression cassette including a nucleotide sequence comprising the HIV antigen-encoding region and the biologically-active component-encoding region according to the present invention for insertion into a plasmid.

A still further aspect of the present invention provides for an expression vector for use in increasing an immune response to HIV in a subject, wherein the expression vector comprises the HIV antigen-encoding region and the biologically-active component-encoding region according to the present invention, which retains some or all of the biological activity of the native domain.

In the following, the present invention is described in more general terms.

### BRIEF DESCRIPTION OF THE DRAWINGS

Figure 1 shows a diagrammatic depiction of a generic DNA vaccine that co-expresses an antigen and an adjuvant, immunoregulatory agent, antisense RNA, or catalytic RNAs through the use of a dicistronic mRNA.
Figure 2 shows diagrammatic depictions of a generic DNA vaccine that co-expresses an antigen and an adjuvant, immunoregulatory agent, antisense RNA, or catalytic RNA each using an independent promoter.
Figure 3 shows the nucleotide sequence of *htat* and the amino acid sequence of *htat*
Figure 4 shows the recombinant DNA strategy used to construct pOGL1-wT.
Figure 5 shows the serum IgG response to HIV-1 gp120 by mice that were vaccinated with pOGL1 and pOGL1-wT that a strong serum IgG response against gp120 was developed, whereas the mice vaccinated with pOGL1-wT did no develop an anti-gp120 IgG response.
Figure 6 shows the results of the ELISPOT assay which indicate that mice vaccinated with plasmid pOGL1 develop a strong gp120-specific IFN-γ-secreting CD8⁺ T cell response, whereas the mice vaccinated with pOGL1-wT did not elicit a measurable gp120-specific IEN-γ-secreting CD8⁺ T cell response.
Figure 7 shows the results of mice challenged with vaccinia-*env* vector strain vT26, which expresses Env of HIV-1_{MN}.
Figure 8 shows the scheme used in the construction of a novel DNA vaccine that co-expresses an antigen (e.g., gp120) and an adjuvant (e.g., CtxA1), referred to herein as "pOGL1-A1", constructed by replacing tat in pOGL-wT with sequences encoding the A1 domain of CT (i.e. CtxA1).
Figure 9 shows data indicating that mice vaccinated with pOGL1-A1 develop a serum IgG response against gp120 that is 10-fold greater than the anti-gp120 serum IgG response in pOGL1-vaccinated mice 22 days after vaccination, when the peak response occurs in these latter mice.
Figure 10 shows data indicating that mice vaccinated with the dicistronic DNA vaccine pOGL1-A1 and the mixture of pOGL1 and pRc/CMV-ctxA1 developed strong serum IgG responses against gp120 that were substantially stronger that the anti-gp120 serum IgG response that arose in pOGL1 vaccinated mice.
Figure 11 shows the recombinant DNA strategy used to incorporate TRP-1 into a constructed pOGL1-wT.
Figure 12 shows the recombinant DNA strategy used to incorporate nucleotide sequences encoding for TRP-1 and TGF-β into a pcDNA3.1.
Figure 13 shows the results of an ELISPOT assay showing that mice vaccinated with plasmid pOGL1, pOGL1-ΔT and pOGL1-pOGL2 developed a strong gp120-specific IFN-γ-secreting CD8⁺ T cell response, whereas mice vaccinated with pOGL1-wT did not elicit a measurable gp120-specific IFN-γ-secreting CD8⁺ T cell response.
Figure 14 is a flow diagram illustrating an algorithm for construction of CED vaccines.

### DETAILED DESCRIPTION OF THE INVENTION

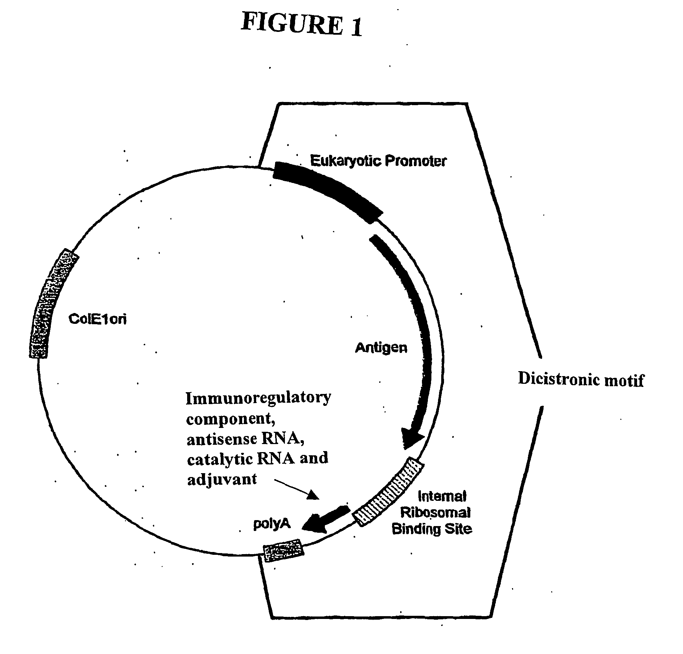

Generic structure of co-expression DNA vaccines: Two preferred configurations are provided as exemplary of the CED vaccines of the present invention. The first preferred configuration expresses a dicistronic mRNA comprising a plasmid backbone, a promoter that is functional in eukaryotic cells, an internal ribosomal entry site additional genetic element, at least one vaccine antigen, and at least one biologically-active component including immunoregulatory proteins and peptides. Further, the CED vaccines of the present invention can encode adjuvants, antisense RNAs and/or catalytic RNAs. Diagrammatic depiction of generic CED vaccines that express dicistronic mRNAs are shown in Figure 1.

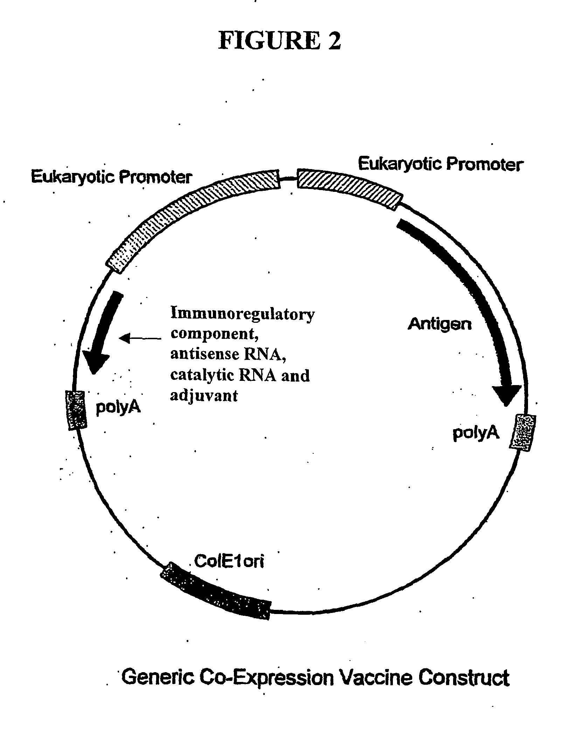

In the second configuration, the CED vaccine expresses at least two products from distinct promoters comprising a plasmid backbone, a promoter that is functional in eukaryotic cells, at least one vaccine antigen, and at least one biologically-active component such as an immunoregulatory protein or peptide. Further, the CED vaccines of the present invention can encode adjuvants, antisense RNAs and/or catalytic RNAs. Diagrammatic depictions of generic CED vaccines that express two mRNAs are shown in Figure 2.

Plasmid vectors useful for co-expression DNA vaccines: The particular plasmid backbone employed in the present invention is not critical thereto, and can be selected from any of the many commercially available cassettes, such as pBR322 (ATCC# 31344); pUC19 (ATCC# 37254); pcDNA3.1_{ZEO} (Invitrogen Cat.# V790-20), pRc/CMV (GenBank accession E14286) obtained from Invitrogen Corporation (San Diego, CA); pXT1 (GenBank accession M26398) or pSG5 (GenBank accession Af013258), obtained from Stratagene (La Jolla, CA); pPUR (GenBank accession U07648) or pMAM (GenBank accession U02443) obtained from ClonTech (Palo Alto, CA); pDual (GenBank accession # AF041247); pG51uc (GenBank accession # AF264724); pACT (GenBank accession # AF264723); pBIND (GenBank accession # AF264722); pCI-Neo (GenBank accession # U47120); pCMV-BD (GenBank accession # AF151088); pIRES-P (GenBank accession # Z75185); pRL-CMV (GenBank accession # AF025843) or synthesized either *denovo* or by adaptation of a publicly or commercially available eukaryotic expression system. Procedures for *de* novo DNA synthesis are described herein below.

Promoters useful for co-expression DNA vaccine: The particular promoter employed in the present invention may be selected from promoters useful for driving expression of genes in animal cells, such as the viral promoters or parts or derivatives thereof, such as the cytomegalovirus immediate early promoter/enhancer (GenBank accession # AF025843) and rous sarcoma virus long terminal repeat promoter (GenBank accession # M83237; Lon, et al., Hum. Immunol., 31: 229-235 (1991)).

Alternatively, the promoter employed in the present invention can be selected from eukaryotic promoters useful for driving expression of genes in animal cells or parts thereof including but not limited to the β-casein promoter (GenBank accession # AF194986; Fan, et al., Direct submission (2000)); uteroglobin promoter (GenBank accession # NM003357; Hay, et al., Am. J. Physiol., 268: 565-575 (1995)); the desmin gene promoter that is only active in muscle cells (Loirat, et al., Virology, 260:74 (1999)); the constitutively expressed β-actin promoter (GenBank accession # NM001101; Vandekerckhove and Weber. Proc. Natl. Acad. Sci. U.S.A., 73: 1106-1110 (1978)); ubiquitin (GenBank accession # AJ243268) or the tyrosinase promoter (GenBank accession # NM000372; Shibaharo, et al., J. Exp. Med., 156: 403-414 (1988)).

Although the particular promoter is not critical to the present, there will be exceptions when the object is to selectively target expression to specific cell types. In this case, the selected promoter is one that is only active in the target cell type. Examples of tissue specific promoters include, but are not limited to, β S1- and β-casein promoters which are specific for mammary tissue (Platenburg, et al., Trans. Res., 3:99-108 (1994); and Maga, et al., Trans. Res., 3:36-42 (1994)); the phosphoenolpyruvate carboxykinase promoter which is active in liver, kidney, adipose, jejunum and mammary tissue (McGrane, et al., J. Reprod. Fert., 41:17-23 (1990)); the tyrosinase promoter which is active in lung and spleen cells, but not testes, brain, heart, liver or kidney (Vile, et al., Canc. Res., 54:6228-6234 (1994)); the involucerin promoter which is only active in differentiating keratinocytes of the squamous epithelia

(Carroll, et al" J. Cell Sci., 103:925-930 (1992)); the uteroglobin promoter which is active in lung and endometrium (Helftenbein, et al., Annal. N.Y. Acad. Sci., 622:69-79 (1991)) and the desmin gene promoter that is only active in muscle cells (Loirat, et al., Virology, 260:74 (1999)).

Internal ribosome entry sites useful for co-expression DNA vaccines: Translation of mRNA in eukaryotic cells requires the presence of a ribosomal recognition signal. Prior to initiation of translation of mRNA in eukaryotic cells, the 5-prime end of the mRNA molecule is "capped" by addition of methylated guanylate to the first mRNA nucleotide residue (Lewin, Genes V, Oxford University Press, Oxford (1994); Darnellet, et al., Molecular Cell Biology, Scientific American Books, Inc., W.H. Freeman and Co., New York, NY (1990)). It has been proposed that recognition of the translational start site in mRNA by the eukaryotic ribosomes involves recognition of the cap, followed by binding to specific sequences surrounding the initiation codon on the mRNA. After recognition of the mRNA by the ribosome, translation initiates and typically produces a single protein species per mRNA molecule (Lewin, Genes V, Oxford University Press, Oxford (1994); Darnell, et al., Molecular Cell Biology, Scientific American Books, Inc., W.H. Freeman and Co., New York, NY (1990)).

It is possible for cap independent translation initiation to occur and/or to place multiple eukaryotic coding sequences within a eukaryotic expression cassette if an internal ribosome entry sequence (IRES) is present on the mRNA molecule (Duke, et al., J. Virol., 66:1602-1609 (1992)). IRES are used by viruses and occasionally in mammalian cells to produce more than one protein species per mRNA molecule as an alternative strategy to mRNA splicing ((Creancier, et al., J. Cell. Biol., 150:275 (2000); Izquierdo and Cuezva, Biochem. J., 346:849 (2000)).

The particular IRES employed in the present invention is not critical and can be selected from any of the commercially available vectors that contain IRES sequences such as those located on plasmids pCITE4a-c (Novagen, http://www.novagen.com); US patent # 4,937,190); pSLIRES11 (Accession: AF171227; pPV (Accession # Y07702); pSVIRES-N (Accession #: AJ000156); Creancier, et al., J. Cell Biol., 10: 275-281 (2000); Ramos and Martinez-Sala, RNA, 10: 1374-1383 (1999); Morgan, et al., Nucleic Acids Res., 20: 1293-1299 (1992); Tsukiyama-Kohara, et al., J. Virol., 66: 1476-1483 (1992); Jang and Wimmer, et al., Genes Dev., 4: 1560-1572 (1990)), on the dicistronic retroviral vector (Accession #: D88622); found in eukaryotic cells such as the Fibroblast growth factor 2 IRES for stringent tissue-specific regulation (Creancier, et al., J. Cell. Biol., 150:275 (2000)) or the Internal-ribosome-entry-site of the 3'-untranslated region of the mRNA for the beta subunit of mitochondrial H⁺-ATP synthase (Izquierdo and Cuezva, Biochem. J., 346:849 (2000)).

Antigens useful for co-expression DNA vaccines: In the present invention, the CED encodes an antigen which may be either a foreign antigen or an endogenous antigen.

As used herein, "foreign antigen" refers to a protein or fragment thereof, which is foreign to the recipient animal cell or tissue including, but not limited to, a viral protein, a parasite protein, an immunoregulatory agent, or a therapeutic agent.

The term "endogenous antigen" is used herein to refer to a protein or part thereof that is naturally present in the recipient animal cell or tissue, such as a cellular protein, a immunoregulatory agent, or a therapeutic agent.

The foreign antigen may be a protein, an antigenic fragment or antigenic fragments thereof that originate from viral and parasitic pathogens.

Alternatively, the foreign antigen may be encoded by a synthetic gene and may be constructed using conventional recombinant DNA methods (See example 1 for synthetic gene construction procedures); the synthetic gene may express antigens or parts thereof that originate from viral and parasitic pathogens. These pathogens can be infectious in humans, domestic animals or wild animal hosts.

The foreign antigen can be any molecule that is expressed by any viral or parasitic pathogen prior to or during entry into, colonization of, or replication in their animal host.

The viral pathogens, from which the viral antigens are derived include, but are not limited to, Orthomyxoviruses, such as influenza virus (Taxonomy ID: 59771); Retroviruses, such as RSV, HTLV-1 (Taxonomy ID: 39015) and HTLV-II (Taxonomy ID: 11909); Herpes viruses, such as EBV (Taxonomy ID: 10295), CMV (Taxonomy ID: 10358) or herpes simplex virus (ATCC #: VR-1487); Lentiviruses, such as HIV-1 (Taxonomy ID: 12721) and HIV-2 Taxonomy ID: 11709); Rhabdoviruses, such as rabies; Picomoviruses, such as Poliovirus (Taxonomy ID: 12080); Poxviruses, such as vaccinia Taxonomy ID: 10245); Rotavirus Taxonomy ID: 10912); and Parvoviruses, such as adeno-associated virus 1 (Taxonomy ID: 85106).

Examples of viral antigens include, but are not limited to, the human immunodeficiency virus antigens Nef (National Institute of Allergy and Infectious Disease HIV Repository Cat. # 183; GenBank accession # AF238278), Gag, Env (National Institute of Allergy and Infectious Disease HIV Repository Cat. # 2433; GenBank accession # U39362), Tat (National Institute of Allergy and Infectious Disease HIV Repository Cat. # 827; GenBank accession # M13137), Rev (National Institute of Allergy and Infectious Disease HIV Repository Cat. # 2088; GenBank accession # L14572), Pol (National Institute of Allergy and Infectious Disease HIV Repository Cat. # 238; GenBank accession # AJ237568) and T cell and B cell epitopes of gp120 (Hanke and McMichael, AIDS Immunol Lett., 66:177 (1999); Hanke, et al., Vaccine, 17:589 (1999); Palker, et al., J. Immunol., 142:3612-3619 (1989)); the hepatitis B surface antigen (GenBank accession # AF043578; Wu, et al., Proc. Natl. Acad. Sci., USA, 86:4726-4730 (1989)); rotavirus antigens, such as VP4 (GenBank accession # AJ293721; Mackow, et al., Proc. Natl. Acad. Sci., USA, 87:518-522 (1990)) and VP7 (GenBank accession # AY003871; Green, et al., J. Virol., 62:1819-1823 (1988)); influenza virus antigens, such as hemagglutinin (GenBank accession # AJ404627; Pertmer and Robinson, Virology, 257:406 (1999)); nucleoprotein (GenBank accession # AJ289872; Lin, et al., Proc. Natl. Acad. Sci., 97: 9654-9658 (2000)); and herpes simplex virus antigens, such as thymidine kinase (GenBank accession # AB047378; Whitley, et al., In: New Generation Vaccines, pages 825-854).

The parasitic pathogens, from which the parasitic antigens are derived, include but are not limited to, *Plasmodium spp.,* such as Plasmodium falciparum (ATCC#: 30145); *Trypanosome spp.,* such as Trypanosoma cruzi (ATCC#: 50797); *Giardia spp.,* such as Giardia intestinalis (ATCC#: 30888D); *Boophilus spp.; Babesia spp.,* such as Babesia microti (ATCC#: 30221); *Entamoeba spp.,* such as Entamoeba histolytica (ATCC#: 30015); *Eimeria spp.,* such as Eimeria maxima (ATCC# 40357); *Leishmania spp.,* (Taxonomy ID: 38568); *Schistosome spp.,* such as Schistosoma mansoni (GenBank accession # AZ301495); *Brugia spp.,* such as Brugia malayi (GenBank accession # BE352806); *Fascida spp.,* such as Fasciola hepatica (GenBank accession # AF286903); *Dirofilaria spp.,* such as Dirofilaria immitis (GenBank accession # AF008300); *Wuchereria spp.,* such as Wuchereria bancrofti (GenBank accession # AF250996); and *Onchocerea spp;* such as Onchocerca volvulus (GenBank accession # BE588251).

Examples of parasite antigens include, but are not limited to, the pre-erythrocytic stage antigens of *Plasmodium spp.* (Sadoff, et al., Science, 240:336-337 (1988); Gonzalez, et al., J. Infect. Dis., 169:927 (1994); Sedegah, et al., Proc. Natl. Acad. Sci., 91:9866 (1994); Gramzinski, et al., Vaccine, 15:913 (1997); Hoffman, et al., Vaccine, 15:842 (1997)), such as the circumsporozoite antigen of *P. falciparum* (GenBank accession # M22982) *P vivax* (GenBank accession # M20670); the liver stage antigens of *Plasmodium spp.* (Hollingdale, et al., Ann. Trop. Med. Parasitol., 92:411 (1998), such as the liver stage antigen 1 (as referred to as LSA-1; GenBank accession # AF086802); the merozoite stage antigens of *Plasmodium spp.* (Holder, et al., Parassitologia, 41:409 (1999); Renia, et al., Infect. Immun., 65:4419 (1997); Spetzler, et al., Int. J. Pept. Prot. Res., 43:351-358 (1994)), such as the merozoite surface antigen-1 (also referred to as MSA-1 or MSP-1; GenBank accession # AF199410); the surface antigens of *Entamoeba histolytica* (Mann, et al., Proc. Natl. Acad. Sci., USA, 88:3248-3252 (1991)), such as the galactose specific lectin (GenBank accession # M59850) or the serine rich *Entamoeba histolytica* protein (also referred to as SREHP; Zhang and Stanley, Vaccine, 18:868 (1999)); the surface proteins of *Leishmania spp.* (also referred to as gp63; Russell, et al., J. Immunol., 140:1274-1278 (1988); Xu and Liew, Immunol., 84: 173-176 (1995)), such as 63 kDa glycoprotein (gp63) of *Leishmania major* (GenBank accession # Y00647 or the 46 kDa glycoprotein (gp46) of *Leïshmania major* (Handman, et al., Vaccine, 18: 3011-3017 (2000); paramyosin of *Brugia malayi* (GenBank accession # U77590; Li, et al., Mol. Biochem. Parasitol., 49:315-323 (1991)); the triose-phosphate isomerase of *Schistosoma mansoni* (GenBank accession # W06781; Shoemaker, et al., Proc. Natl. Acad Sci., USA, 89:1842-1846 (1992)); the secreted globin-like protein of *Trichostrongylus colubriformis* (GenBank accession # M63263; Frenkel, et al., Mol. Biochem. Parasitol., 50:27-36 (1992)); the glutathionerS-transferases of *Fasciola hepatica* (GenBank accession # M77682; Hillyer, et al., Exp. Parasitol, 75:176-186 (1992)); *Schistosoma bovis* (GenBank accession # M77682); *S. japonicum* (GenBank accession # U58012; Basbir, et al., Trop. Greog. Med., 46:255-258 (1994)); and KLH of *Schistosoma bovis* and *S. japonicum* (Bashir, et al., *supra*),

As mentioned earlier, the CED vaccines may encode an endogenous antigen, which may be any cellular protein, immunoregulatory agent, or therapeutic agent, or parts thereof, that may be expressed in the recipient cell including, but not limited to, tumor, transplantation and autoimmune antigens, or fragments and derivatives of tumor, transplantation and autoimmune antigens thereof. Thus, the CED vaccine may encode tumor, transplant, or autoimmune antigens or parts or derivatives thereof. Alternatively, the CED vaccine may encode synthetic genes, which encode tumor-specific, transplant, or autoimmune antigens or parts thereof.

Examples of tumor specific antigens include prostate specific antigen (PSA) (Gattuso, et al., Human Pathol., 26:123-126 (1995)), TAG-72 and CEA (Guadagai, et al., Int. J. Biol. Markers, 9:53-60 (1994)); human tyrosinase (GenBank accession # M27160; Drexler, et al., Cancer Res., 59:4955 (1999); Coulie, et al., J. Immunothera, 14:104-109 (1993)); tyrosinase-related protein (also referred to as TRP; GenBank accession # AJ132933; Xiang, et al., Proc. Natl. Acad Sci., 97:5492 (2000)); and tumor-specific peptide antigens (Dyall, et al., J. Exp. Med., 188:1553 (1998).

Examples of transplant antigens include the CD3 molecule on T cells (Alegre, et al., Digest, Dis. Sci., 40:58-64 (1995)) and histocompatibility antigens such as HLA A, HLA B, HLA C, HLA. DR and HLA DQ (Janeway, et al., In: Immunobiology: The immune system in health and disease (Fourth Edition), Current Biology Publications, London W1P 6LB, United Kingdom, Ch. 13, pp509-519 (1999)).

Examples of autoimmune antigens include IAS β chain, which is useful in therapeutic vaccines against autoimmune encephalomyetitis (GenBank accession # D88762; Topham, et al., Proc. Natl. Acad. Sci., 91:8005-8009 (1994)); glatamic acid decarboxylase, which is useful in therapeutic vaccines against insulin-dependent type 1 diabetes (GenBank accession # NM013445; Quin, et al., J. Autoimmun., 11:591 (1998)); thyrotropin receptor (TSHr), which is useful in therapeutic vaccines against Grave's disease (GenBank accession # NM000369; Costagliola, et al., J. Clin. Invest, 105:803 (2000)) and tyrosinase-related protein 1, which is useful in therapeutic vaccines against vitiligo (GenBank accession # NM000550; Overwijk, et al., Proc. Natl. Acad, Sci., 96:2982 (1999)).

Immunogulatory molecules useful for co-expression DNA vaccines: In the present invention, in addition to encoding an exogenous or endogenous antigen, the CED vaccines can also encode a biologically-active component including an immunoregulatory molecule. A diagrammatic depiction of a generic CED vaccine encoring a vaccine antigen and an immunoregulatory molecule on a dicistronic DNA expression cassette is shown in Figure 1. A diagrammatic depiction of a generic CED vaccine encoding a vaccine antigen and an immunoregulatory molecule that are expressed by separate promoters is shown in Figure 2.

Examples of immunoregulatory molecules may include:
- growth factors, such as TGF-β (GenBank accession # Q99988), monocyte colony stimulating factor (M-CSF, GenBank accession # E02187), granulocyte-monocyte colony stimulating factor (GM-CSF, GenBank accession # M14663);
- cytokines (e.g., GenBank accession # U31279), such as interleukin-2 (IL-2, GenBank accession # AF031845), interleukin-4 (IL4, GenBank accession # M13982), interleukin-5 (IL-5, GenBank accession # AF051372), interleukin-6 (IL-6, GenBank accession # NM002184), interleukin-10 (IL-10, GenBank accession # X78437), interleukin-12 (IL-12, GenBank accession # AF180563); interleukin-18 (IL-18, GenBank accession # NM 001562); interferon-gamma inducible protein (GenBank accession # BE501937); and interferon-gamma (IFN-γ, GenBank accession # AW079182);
- chemokines, such as macrophage inhibitory protein-1 alpha (MIP-1α, GenBank accession # A34528), macrophage inhibitory protein-1 beta (MIP-1β, GenBank accession # AF031587), macrophage inhibitory protein-3 alpha (MIP3α, GenBank accession # A17117), monocyte derived chemokine (MDC, GenBank accession # AF076596), RANTES (GenBank accession # AF266753), interleukin-8 (IL-8, GenBank accession # S78555), and stromal cell derived factor-1alpha (SDF-1α, GenBank accession # AF189724);
- co-stimulatory molecules, such as CD80 (GenBank accession # NM005191); CD86 (GenBank accession # NM019388); cytotoxic T-lymphocyte-associated protein-4 (CTLA-4, GenBank accession # AH002733); CD28 (GenBank accession # NM006139); CD40 (GenBank accession # Y10507); and CD40 ligand (GenBank accession # D31793);
- cytokine receptors such as the alpha chain of the interferon-gamma receptor (GenBank accession # AW771757); the beta chain of the interferon-gamma receptor (GenBank accession # AW771346);
- viral immunoregulatory molecules, such as the Tat protein of human immunodeficiency virus-1 (HIV-1, GenBank accession # U39362), HIV-2 (GenBank accession # AF208027), simian immunodeficiency virus (SIV, GenBank accession # U42720), or feline immunodeficiency virus (FIV, GenBank accession # NC 001482; Albott, et al., Proc. Natl. Acad. Sci., 86:5743 (1989)), the Tax protein of human T-cell lymphotropic virus type 1 (HTLV-1, GenBank accession # NC 001436) and HTLV-II (GenBank accession # NC 001488); and
- bacterial toxins that up-regulate cAMP levels, such as the A subunit (referred to herein as CtxA) of cholera toxin (GenBank accession # D30052 and D30053), the A subunit of heat-labile toxin (referred to herein as EltA) of enterotoxigenic *Escherichia coli* (GenBank accession # M35581); pertussis toxin S1 subunit (PtxS1, GenBank accession # AJ007364, AJ007363, AJ006159, AJ006157, etc.), and adenylcyclase toxin of *Bordetella pertussis* (ATCC # 8467) or *Bordetella bronchiseptica* (ATCC # 7773).

Antisense_RNA useful for co-expression DNA vaccines: In addition to encoding a vaccine antigen, the CED vaccine can also express antisense RNA. A diagrammatic depiction of a generic CED vaccine encoding a vaccine antigen and an antisense RNA on a dicistronic DNA expression cassette is shown in Figure 1. A diagrammatic depiction of a generic CED vaccine encoding a vaccine antigen and an antisense RNA that are expressed by separate promoters is shown in Figure 2.

Particular antisense RNAs include antisense RNAs that target molecules present within the recipient cell, including but not limited to RNA species encoding the cell immunoregulatory molecules, such as described hereinabove.

Catalytic RNA useful for co-expression DNA vaccines: In addition to encoding a vaccine antigen, the CED vaccine can also express catalytic RNA. A diagrammatic depiction of a generic CED vaccine encoding a vaccine antigen and a catalytic RNA on a dicistronic DNA expression cassette is shown in Figure 1. A diagrammatic depiction of a generic CED vaccine encoding a vaccine antigen and a catalytic RNA that are expressed on separate DNA expression cassettes is shown in Figure 2.

Particular catalytic RNAs include catalytic RNAs that target molecules present within the recipient cell. These include but are not limited to RNA species encoding cell immunoregulatory molecules, such as described hereinabove.

DNA vaccines useful for a murine model of Tat-mediated immune deviation: Particular DNA vaccines are suitably configured using at least one of three following configurations:
The first configuration utilizes a DNA vaccine that expresses a dicistronic mRNA and generally comprises a plasmid vector, a promoter that is functional in eukaryotic cells, an internal ribosornal entry site additional genetic elements, an experimental antigen (e.g. HIV-1 antigens such as the Env, gp120, gp120, Pol, Gag, Nef, or Rev proteins), and the HIV-1 Tat protein. Diagrammatic depiction of a generic CED vaccine for expressing a dicistronic mRNA is shown in Figure 1.

In a second configuration, the DNA vaccine that expresses at least two products, one is an experimental antigen (e.g. HIV-1 antigens such as the Env, gp120, gp120, Pol, Gag, Nef, or Rev proteins), and the other is the HIV-1 Tat protein; the expression in this configuration occurs from distinct promoters as shown in Figure 2.

In a third configuration, two DNA vaccines are used as a mixture, one that expresses an experimental antigen (e.g. HIV-1 antigens such as the Env, gp120, gp120, Pol, Gag, Nef, or Rev proteins) and another that expresses the HIV-1 Tat protein.

Examples of suitable sources include HIV-1, HIV-2 or SIV (AIDS Repository, National Institute of Allergy and Infectious Disease, Bethesda MD). Alternatively a synthetic Tat gene (*htat*) can be constructed that is optimized for expression in mammalian cells using any of the aforementioned HIV-1, HIV-2 or SIV Tat amino acid sequences as blueprints and the codon replacement strategy described elsewhere (Haas, et al., Curr. Biol., 6:315-24 (1996); Andre, et al., J. Virol., 72:1497-503 (1998)). The plasmid vectors, promoters, and IRES may be any of those set forth above.

Generic structure of DNA vaccines that co-express an antigen and an adjuvant: The particular novel DNA vaccines, which co-express an antigen and an adjuvant, employed in the present invention can be readily synthesized and formulated, preferably using one of the following two preferred configurations.

The first preferred configuration of a DNA vaccine that co-expresses an antigen and an adjuvant utilizes a dicistronic mRNA and generally comprises a plasmid vector, a promoter that is functional in eukaryotic cells, an internal ribosomal entry site, additional genetic elements, at least one vaccine antigen, and an bacterial toxin that augments cAMP levels (e.g., a member of the family of bacterial adenosine diphosphate-ribosylating exotoxins (i.e. CtxA of Vibrio choleraited, EltA of *Escherichia coli*). Diagrammatic depiction of a generic DNA vaccine that co-expresses an antigen and an adjuvant through the use of a dicistronic mRNA is shown in Figure 1.

In the second configuration, the DNA vaccine that co-expresses an antigen and an adjuvant utilizes two promoters and is composed of a plasmid vector, two promoters that are functional in eukaryotic cells, at least one antigen, and an bacterial toxin that augments cAMP levels (e.g., a member of the family of bacterial adenosine diphosphate-ribosylating exotoxins (i.e. CtxA of Vibrio cholera or EltA of *Escherichia coli*); an adenylate cyclase-hemolysin (i.e. CyaA of *Bordetella spp*)). Diagrammatic depictions of a generic DNA vaccine that co-expresses an antigen and an adjuvant that utilizes two promoters is shown in Figure 2.

The particular adjuvant that constitutively augments cAMP levels may be the A subunit of cholera toxin (i.e. CtxA; GenBank accession no. X00171, AF175708, D30053, D30052,), or parts thereof (e.g., the A1 domain of the A subunit of Ctx (i.e. CtxA1; GenBank accession no. K02679)), from any classical *Vibrio cholerae* (e.g., *V. cholerae* strain 395, ATCC # 39541) or E1 Tor *V. cholerae* (e.g., *V. cholerae* strain 2125, ATCC # 39050) strain. Alternatively, any bacterial toxin that increases cellular cAMP levels, such as a member of the family of bacterial adenosine diphosphate-ribosylating exotoxins (Krueger and Barbier, Clin. Microbiol. Rev., 8:34 (1995)), may be used in place of CtxA, for example the A subunit of heat-labile toxin (referred to herein as EltA) of enterotoxigenic *Escherichia coli* (GenBank accession # M35581), pertussis toxin S1 subunit (e.g., *ptxS1,* GenBank accession # AJ007364, AJ007363, AJ006159, AJ006157, etc.). As another example, the adjuvant may be one of the adenylate cyclase-hemolysins of *Bordetella pertussis* (ATCC # 8467), *Bordetella bronchiseptica* (ATCC # 7773) or *Bordetella parapertussis* (ATCC # 15237), e.g., the *cyaA* genes of *B. pertussis* (GenBank accession # X14199), *B. parapertussis* (GenBank accession # AJ249835) or *B. bronchiseptica* (GenBank accession # Z37112).

Genetic engineering procedures and reagents for the preparation of co-expression DNA vaccines: The construction of CED vaccines is accomplished using an assortment of well-known recombinant DNA procedures.

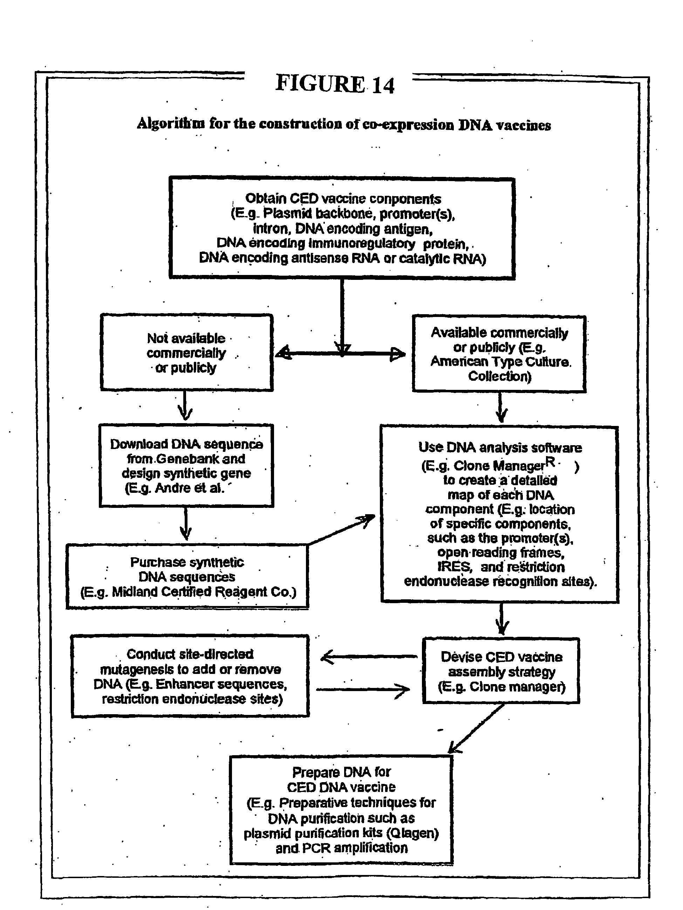

Algorithm for the construction of co-expression DNA vaccines: As mentioned earlier, the components of the CED vaccines of the present invention comprise a plasmid backbone carrying a multi-component DNA insert comprising at least one promoter, DNA encoding the first protein product, DNA encoding the second protein product, and an IRES (e.g., In instances when the protein products are expressed from dicistronic mRNA). Due to the requirement of components that are not always available from a single source, an algorithm for the assembly of the pertinent components in shown in Figure 14 that illustrates isolation of the individual DNA components, construction, purification and formulation of the CED vaccine.

Recombinant DNA methods: The CED vaccines described herein are produced using procedures well known in the art. Examples include polymerase chain reaction (PCR; Sambrook, et al., Molecular cloning; A laboratory Manual: Vol. 1-3, Cold Spring Harbor Laboratory Press, Cold Spring Harbor, New York (1989)); DNA synthesis using an Applied Biosystems DNA synthesizer (Perkin Elmer ABI 3948, using the standard cycle as according to procedures provided by the manufacturer); agarose gel electrophoresis (Ausubel, Brent, Kingston, Moore, Seidman, Smith and Struhl, Current Protocols in Molecular Biology: Vol. 1 and 2, Greene Publishing Associates and Wiley-Interscience, New York (1990)); restriction endonuclease digestion of DNA (Sambrook, et al., *supra* (1989)); annealing DNA fragments using bacteriophage T4 DNA ligase (New England Biolabs, Cat #202CL; Sambrook, Fritsch, and Maniatis. Molecular cloning; A laboratory Manual: Vol. 1-3, Cold Spring Harbor Laboratory Press, Cold Spring Harbor, New York (1989)); introducing recombinant plasmids into *Escherichia coli* by electrotransformation (also called electroporation; (Sambrook, et al., *supra* (1989)); culturing of *E. coli* isolates that carry recombinant plasmids on solid media (e.g., Tryptic Soy Agar; Beckton Dickenson, Sparks, MD cat #211046) or in liquid media (e.g., Tryptic Soy Broth; Beckton Dickenson, Sparks, MD cat #211771) containing the appropriate antibiotics (e.g., 100 µg/ml ampicillin 20 µg/ml chloramphenicol or 50 µg/ml kanamycin) for the selection of bacteria that carry the recombinant plasmid; isolation of plasmid DNA using commercially available DNA purification kits (Qiagen, Santa Clarita, CA EndoFree Plasmid Maxi Kit, cat # 12362); transfection of murine and human cells using the FuGENE^{R} proprietary multi-component transfection system using the procedure recommended by the manufacturer (Roche Diagnostics Corporation, Roche Molecular Biochemicals, Indianapolis, in cat # 1815091; e.g., Schoonbroodt and Piette, Biochemica, 1:25 (1999)); culturing murine or human cells lines in RPMI 1640 medium (Life Technologies, Gaithersburg MD) containing 10% (v/v) fetal calf serum (Gemini Bioproducts, Calabasas, CA. cat #100-107; See also Current Protocols in Immunology); analysis of tissue culture supernatants and cell lysates by sodium dodecylsufate-polyacrylamide gel electrophoresis (SDS-PAGE; Harlow and Lane. Using Antibodies, A Laboratory Manual, Cold Spring Harbor Laboratory Press, NY, (1988)) and immunoblotting (Harlow and Lane. Using Antibodies, A Laboratory Manual. Cold Spring Harbor Laboratory Press, NY, (1988)); quantitation of recombinant proteins produced by recombinant plasmids in murine or human cells using quantitative immunoblot, capture enzyme-linked immunosorbent assays (ELISAs; Ausubel, et al., Current Protocols in Molecular Biology: Vol. 1 and 2, Greene Publishing Associates and Wiley-Interscience, New York (1990)); alternatively the capture ELISAs can be conducted by a commercial facility such as the U-Quant Facility at the IHV, UMBI, MD, which used Endogen and R&D Systems quantitative ELISA products. (E.g." mIFN ELISA kit cat #EM-1001-1 from Endogen, Woburn, MA. mIL4 ELISA kit cat #M4000, mIL5 ELISA kit cat # M5000, mIL10 ELISA kit cat #M1000, from R & D Systems, Minneapolis, MN), or quantitative reverse transcriptase (RT)-PCR (Ausubel, et al., IN: Current Protocols in Molecular Biology: Vol. 1 and 2, Greene Publishing Associates and Wiley-Interscience, New York (1990)), using the Thermoscript RT-PCR System (Life Technologies, Gaithersburg MD; cat #11146-016).

Generation of specific DNA sequences: DNA sequences encoding the individual elements of CED vaccines, such as the promoter, enhancer, genetic element, vaccine antigen, internal ribosome entry site (IRESs), immunoregulatory protein, antisense RNA and catalytic RNA, can be obtained from the American Type Culture Collection (ATCC, Manassas, VA). Bacteria containing the recombinant plasmid that carries the sequence of interest are cultured, the plasmid DNA is purified and the target sequence is isolated by using restriction endonucleases or by PCR amplification as described hereinbelow.

Alternatively, in instances where the desired DNA sequence is not available at the ATCC, individual DNA sequences can be made *de novo* using the DNA sequence from GenBank or from commercial gene databases, e.g., Human Genome Sciences (Gaithersburg, MD), as the blueprint of the target gene, DNA fragment, or parts thereof. Thus, *de novo*-generated DNA encoding promoters, enhancers, genetic elements, vaccine antigens, internal ribosome entry sites (IRESs), immunoregulatory proteins, antisense RNA and catalytic RNA are created by first synthesizing 100 to 200 nucleotide overlapping oligonucleotides that are subsequently annealed to form double stranded DNA and joined by ligation to form a larger fragment. The joined fragments are then purified and joined by ligation to yet another joined fragment to create larger fragments and so on until the full-length DNA molecule is created. After each round of ligation the joined fragments can be amplified by PCR to increase the yield of the joined fragments. Procedures for *de novo* DNA synthesis are well known to the art and are described elsewhere (Andre, et al., *supra,* (1998); Haas, et al., *supra,* (1996)); alternatively synthetic designer genes can be purchased commercially, e.g., from the Midland Certified Reagent Co. (Midland, TX). Following completion of the *de novo* gene synthesis the integrity of the coding sequence in the resultant DNA fragment is verified by automated dideoxynucleic acid sequencing at a facility that has the appropriate capabilities and equipment, such as the Biopolymer Core Facility, University of Maryland, Baltimore MD.

Purification of co-expression DNA vaccines: The specific method used to purify the CED vaccines of the present invention are not critical and may be selected from previously described procedures used to purify conventional DNA vaccines (e.g., endotoxin-free large-scale DNA purification kits from Qiagen, Santa Clarita, CA; "EndoFree Plasmid Maxi Kit", cat # 12362), or two rounds of purification using Cesium chloride density gradients (Ausubel, et al., *supra* (1990)). Alternatively, purified lots of CED vaccines can be obtained from commercial sources that have the capacity to produce endotoxin-free plasmid DNA preparations using the Good Manufacturing Procedures as outlined by the US Food and Drug Administration (e.g., Ameba lysate agglutination assay, Schwartzman assay in New Zealand White Rabbits).

Formulation of co-expression DNA vaccines: The specific method and reagents used to formulate the CED vaccines described herein is not critical to the present invention. Examples include formulations that combine the CED vaccine with a physiological buffer (Felgner, et al., US Patent # 5589466 (1996)); aluminum phosphate or aluminum hydroxyphosphate (e.g., Ulmer, et al., Vaccine, 18:18 (2000)), monophosphoryl-lipid A (also referred to as MPL or MPLA; Schneerson, et al., J. Immunol., 147: 2136-2140 (1991); e.g., Sasaki, et al., Inf. Immunol., 65: 3520-3528 (1997); Lodmell, et al., Vaccine, 18: 1059-1066 (2000)), QS-21 saponin (e.g., Sasaki, et al., J. Virol., 72:4931 (1998)); dexamethasone (e.g., Malone, et al., J. Biol. Chem., 269:29903 (1994)); CpG DNA sequences (Davis, et al., J. Immunol., 15:870 (1998)); lipopolysaccharide (LPS) antagonist (e.g., Shata and Hone, PCT/00US/27402), a second plasmid encoding a cytokine (e.g., Hayashi, et al., Vaccine, 18: 3097-3105 (2000); Sin, et al., J. Immunol., 162: 2912-2921 (1999); Gabaglia, et al. J. Immunol., 162: 753-760 (1999); Kim, et al., Eur J Immunol., 28:1089 (1998); Kim, et al., Eur. J. Immunol., 28:1089 (1998); Barouch, et al., J. Immunol., 161:1875 (1998); Okada, et al., J. Immunol., 159:3638 (1997); Kim, et al., J. Virol., 74:3427 (2000)), or a second plasmid encoding a chemokine (e.g., Boyer, et al., Vaccine, 17(Suppl 2):S53 (1999); Xin, et al., Clin. Immunol., 92:90 (1999)).

Vaccination strategies: The CED vaccine can be introduced into the animal by intravenous, intramuscular, intradermal, intraperitoneally, intranasal and oral inoculation routes. The specific method used to introduce the CED vaccines described herein into the target animal is not critical to the present invention and can be selected from conventional DNA vaccination methods for intramuscular, intravenous, intradermal, intraperitoneally, and intranasal routes of inoculation (an extensive database of publications describing the above cited vaccination procedures is located at www.DNAvaccine.com/Biblio/articles.html).

Oral inoculation of the target animal with the CED vaccines of the present invention can be achieved using an non-pathogenic or attenuated bacterial DNA vaccine vector (Powell, et al., US Patent No. 5,877,159 (1999)). The amount of the bacterial DNA vaccine vector of the present invention to be administered will vary depending on the species of the subject, as well as the disease or condition that is being treated. Generally, the dosage employed will be about 10³ to 10¹¹ viable organisms, preferably about 10⁵ to 10⁹ viable organisms.

The bacterial DNA vaccine vector carrying the CED vaccine of the present invention is generally administered along with a pharmaceutically acceptable carrier or diluent. The particular pharmaceutically acceptable carrier or diluent employed is not critical to the present invention. Examples of diluents include a phosphate buffered saline, buffer for buffering against gastric acid in the stomach, such as citrate buffer (pH 7.0) containing sucrose, bicarbonate buffer (pH 7.0) alone (Levine, et al., J. Clin. Invest, 79:888-902 (1987); and Black, et al., J. Infect. Dis., 155:1260-1265 (1987)), or bicarbonate buffer (pH 7.0) containing ascorbic acid, lactose, and optionally aspartame (Levine, et al., Lancet, II:467-470 (1988)). Examples of carriers include proteins, e.g., as found in skim milk, sugars, e.g., sucrose, or polyvinylpyrrolidone. Typically these carriers would be used at a concentration of about 0.1-90% (w/v) but preferably at a range of 1-10% (w/v).

The following examples are provided for illustrative purposes only, and are in no way intended to limit the scope of the present invention.

### Example 1

### Recombinant DNA procedures

### Regents, bacterial strains and plasmids

Restriction endonucleases (New England Biolabs Beverly, MA), T4 DNA ligase (New England Biolabs Beverly, MA) Taq polymerase (Life technologies, Gaithersburg, MD) were used according to the manufacturers' protocols. Plasmid DNA was prepared using small-scale (Qiagen Miniprep kit) or large-scale (Qiagen Maxiprep kit) plasmids DNA purification kits according to the manufacturer's protocols (Qiagen, Santa Clarita, CA). Nuclease-free, molecular biology grade milli-Q water, Tris-HCl (pH 7.5), EDTA pH 8.0, 1M MgCl₂, 100% (v/v) ethanol, ultra-pure agarose, and agarose gel electrophoresis buffer were purchased from Life technologies, Gaithersburg, MD. DNA ligation reactions and agarose gel electrophoresis were conducted according to well-known procedures (Sambrook, et al., *supra* (1989); Ausubel, et al., *supra* (1990)).

PCR primers were purchased from the University of Maryland Biopolymer Facility (Baltimore, MD) and were synthesized using an Applied Biosystems DNA synthesizer (model 373A). PCR primers were used at a concentration of 200 µM and annealing temperatures for the PCR reactions were determined using Clone manager software. PCRs were conducted in a Strategene Robocycler, model 400880 (Strategene, La Jolla, CA). Annealing, elongation and denaturation times in the PCRs were set according to well-known procedures.

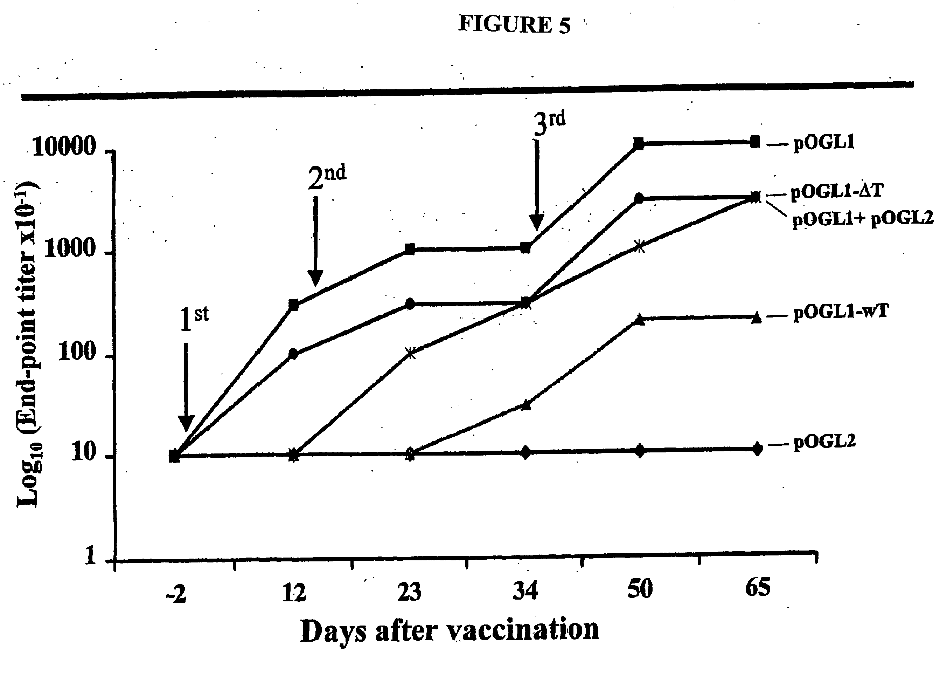

A synthetic *tat* gene (*htat*), optimized for expression in mammalian cells and encoding the HIV-1_{MN} Tat protein was purchased from Midland Certified Reagent Company (Midland, TX). The codon usage pattern was optimized for expression of the Tat-encoding synthetic DNA in mammalian cells, as reported previously (Haas, et al., Curr. Biol., 6: 315-24 (1996); Andre, et al., J Virol., 72: 1497-503 (1998)). The synthetic nucleotide sequence and amino acid sequence of *htat* are shown in Figure 5. The DNA encoding the synthetic, codon-optimized HIV-1_{MN} Tat gene was amplified using forward primer 5'-GCCAAATACATGGCCATTGAGCCCGTGGACCCTCGCCTGGAGCCCT (SEQ ID NO: 19) and reverse primer 5'-ATAAGAATCTCGAGCAGCTGGAATTCGCGGCCGGCTGATCAG (SEQ ID NO: 20).

Nucleotide sequencing to verify the DNA sequence of each recombinant plasmid described in the following examples was accomplished by conventional automated DNA sequencing techniques using an Applied Biosystems automated sequencer, model 373A.

*Escherichia coli* strain Stable2^{™} served as host of the recombinant plasmids described in the examples below. Wild type *Vibrio cholerae* served as a source of DNA sequences encoding the A1 domain of the A subunit of cholera toxin and was provided by the Department of Microbiology and Immunology, University of Maryland, Baltimore.

Recombinant plasmids were introduced into *E. coli* strain Stable2^{™} by electroporation using a Gene Pulser (BioRad Laboratories, Hercules, CA) set at 200Ω, 25 µF and 2.5 kV as described (Hone, et al., Vaccine, 9: 810-816 (1991)).

All bacteria were grown at 37°C on tryptic soy agar or in tryptic soy broth, unless stated otherwise. When appropriate, the media were supplemented with 100 µg/ml ampicillin (Sigma, St. Louis, MO).

Bacterial strains were stored at -80°C suspended in tryptic soy broth containing 30% (v/v) glycerol at *ca*. 10⁹ colony-forming units (cfus) per ml.

Plasmid pCITE4a, which contains the IRES of equine encephalitis virus, was purchased from Novagen (Madison WI).

Plasmid pcDNA3.1_{ZEO}, which contains the colE1 replicon, an ampicillin-resistance allele, the CMV immediate-early promoter, a multicloning site and the bovine hemoglobin poly-adenosine sequence, was purchased from Clonetech (Clonetech, Palo Alto, CA).

Plasmid pEF1a-syngp120_{MN} carrying synthetic DNA encoding HIV-1_{MN} gp120 (referred to herein as *hgp120*), in which the native HIV-1 leader peptide is replaced by the human CD5 leader peptide and the codons are optimized for expression in mammalian cells is described elsewhere (Andre, et al., *supra,* (1998); Haas et al., *supra,* (1996)).

Restriction endonuclease digestion, ligation, and plasmid DNA preparation techniques were all conducted as described hereinbelow.

### Example 2

### Construction of a co-expression DNA vaccine encoding a vaccine antigen and an immunoregulatory protein

Source of DNA sequences: Construction of a conventional DNA vaccine, pOGL1, was achieved by PCR-amplifying *hgp120* from a plasmid pEF1a-syngp120MN (Andre, et al., *supra,* (1998); Haas et al., *supra,* (1996)) using forward primer 5'-GGGGGGGGATCCATGCCCATGGGGTCTCTGCAACCGCTG (SEQ ID NO: 14) and reverse primer 5'-GGGGGCGGCCGCTTATTAGGCGCGCTTCTCGCGCTGCACCACGCG (SEQ ID NO: 15). The resultant PCR-generated DNA fragment was digested with restriction endonucleases *Bam*HI and *Not*I and annealed (E.g., by ligation with T4 ligase) with plasmid pcDNA3.1_{ZEO} (Invitrogen, Carlsbad, CA, Cat. No. V860-20), which had been digested with the identical restriction endonucleases. The annealed chimeric plasmid DNA was introduced into *E. coli* strain Stable2 by electroporation and positive clones were identified by screening small-scale plasmid DNA preparations isolated from individual clones for the appropriate restriction endonuclease digestion patterns. An isolate, referred to herein as strain H1058, containing the plasmid referred to herein as pOGL1, which is pcDNA3.1_{ZEO} containing the *Bam*HI-*Not*I *hgp120* fragment, was stored at -80°C. Additional analysis by restriction endonuclease digestion, PCR of the *hgp120* DNA, and dideoxynucleotide sequencing of the cloned *hgp120* DNA in pOGL1 was conducted to verify that the *hgp120* DNA was not altered during construction.

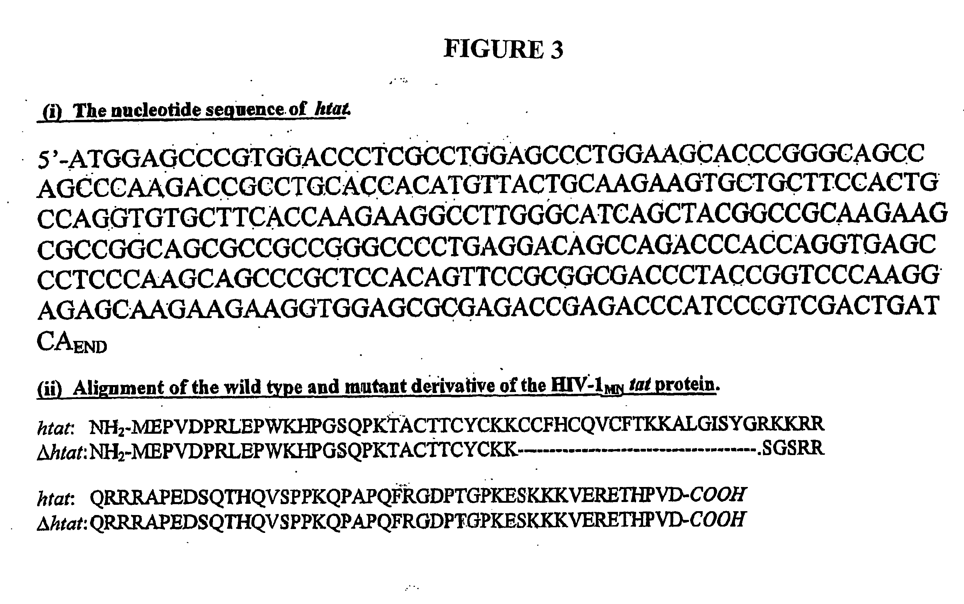

Construction of a synthetic codon optimized Tat allele: A synthetic Tat gene (*htat*) optimized for expression in mammalian cells was designed using the HIV-1_{MN} *tat* amino acid sequence as a blueprint (GenBank accession no. AR034234) and the codon replacement strategy described elsewhere (Haas, et al., Curr. Biol., 6:315-24 (1996); Andre, et al., J Virol., 72, 1497-503 (1998)). The nucleotide sequence of *htat* is shown in Figure 3 (SEQ ID NO: 18). A 306 bp synthetic double stranded DNA fragment encoding *htat* was purchased from Midland Certified Reagent Co. (Midland, TX); the synthetic fragment encodes the complete (e.g., first and second exons) 102 amino acid HIV-1_{MN} *tat* open reading frame with *Bam*HI and *Eco*RI restriction endonuclease sites incorporated into the 5-prime and 3-prime ends, respectively.

A plasmid that expresses HIV-1 Tat protein alone, referred to herein as pOGL2, was constructed by inserting *htat* into the plasmid pcDNA3.1_{ZEO}. Briefly, the synthetic *htat* DNA was digested with restriction endonucleases *Bam*HI and *Eco*RI and annealed to *Bam*HI-, *Eco*RI-digested pcDNA3.1_{ZEO} using T4 DNA ligase.

DNA encoding the IRES of equine encephalitis virus, referred to as the cap-independent translational enhancer (CITE), was amplified by PCR from purified plasmid pCITE4a (Novagen, Madison WI) DNA using forward primer 5'-ATAAGAATGCGGCCGCTAAGTAAGTAACTTAAGTTCCGGTTATTTTCCACG ATATTGCCGTCTTTTGGCAA (SEQ ID NO: 16) and reverse primer 5'-GCCAAATACATGGCCATATTATCATCGTGTTTTTCAAAGGAA (SEQ ID NO: 17).

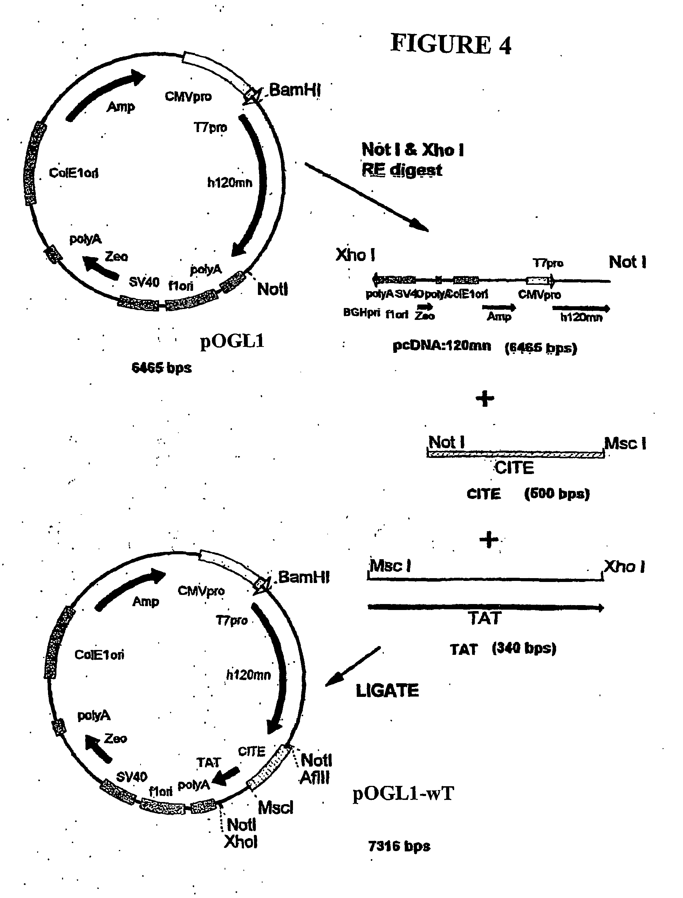

Co-expression DNA vaccine construction strategy: A CED vaccine that expresses the HIV-1_{MN} gp120 and Tat proteins, referred to herein as pOGL1-wT, was constructed by three-way ligation of *hgp120,* CITE, and *htat* DNA as shown in Figure 4. First, DNA encoding CITE was amplified by PCR from plasmid pCITE4a and digested with *Not*I and *Msc*I and the CITE-encoding fragment was separated from the remainder of the vector using agarose gel electrophoresis (Life Technologies, Gaithersburg, MD) and purified using Qiagen gel extraction kit (Qiagen, Valencia, CA). The *htat* gene was isolated from *Msc*I- and *Xho*I-digested pOGL2 DNA using the same purification strategy. Plasmid pOGL1 was digested with *Not*I and *Xho*I. The purified CITE and *htat* DNA fragments were then mixed with *Not*I-digested pOGL1 DNA at a molar ratio of 1:1:1 and the resulting mixture was annealed by ligation and introduced into strain Stable2 by electroporation. Single colony isolates were screened for the presence of recombinant plasmids with the appropriate molecular structure by isolating plasmid DNA and analyzing it by restriction endonuclease digestion and PCR; an isolate containing the appropriate recombinant plasmid, referred to herein as "pOGL1-wT", was identified and the strain harboring pOGL1-wT, referred to herein as "T128" was stored at -80°C. Dideoxynucleotide sequencing of the gp120-CITE-Tat-encoding DNA in pOGL1-wT was conducted to verify that it was not altered during construction.

### Example 3

### Induction of systemic tolerance with a co-expression DNA vaccine encoding a vaccine antigen and an immunoregulatory protein

Vaccination of mice and induction of immune deviation: To investigate the properties of the Tat containing DNA vaccines including, pOGL1, pOGL1-wT and pOGL2, a comparative study was conducted in which the immunogenicity of gp120 expressed by plasmids pOGL1 and pOGL1-wT was assessed in BALB/c mice. To assure that differences in immunogenicity were not due to gp120 expression differences, the level of gp120 expression by pOGL1, pOGL2 and pOGL1-wT was assessed in transiently transfected BALB/c P815 cells and C57BL/6; the expression levels were found to be indistinguishable (300 - 500 ng per 10⁶ cells).

Source of laboratory animals and handling: BALB/c and C57B1/6 mice aged 6-8 weeks were certified to be specific pathogen free and upon arrival at the University of Maryland Biotechnology Institute Animal Facility were maintained in a microisolator environment and allowed to fee and drink *ad lib.*

An immunogenicity study was conducted using 4 groups of 6 BALB/c mice that were vaccinated intramuscularly with three 100 µg-doses of endotoxin-free plasmid DNA suspended in saline (0.85% (w/v) NaCl) at weekly intervals. A fourth 100 µg was injected intramuscularly 28 days after the third dose. In parallel, a negative control group of 6 BALB/c mice received four 100 µg-doses of plasmid pcDNA3.1_{ZEO} DNA using the same protocol.

**TABLE 1**

| **Group** | **Vaccine** | **Relevant genotype** |
|---|---|---|
| 1 | pOGL2 | *htat*⁺ |
| 2 | pOGL1 | *hgp120*⁺ |
| 3 | pOGL1-wT | *hgp120*⁺ and *htat*⁺ (Dicistronic) |
| 4 | pOGL1 + pOGL2 | *hgp120*⁺ and *htat*⁺ (Mixture) |

Serum enzyme-linked immunosorbent assays (ELISAs): Blood (ca. 100 µl per mouse) was collected before and at weekly intervals after vaccination. The presence of gp120-specific IgG in the sera of the vaccinated mice was determined by ELISA. Aliquots (0.3 µg suspended in 100 µl PBS, pH 7.3) of purified glycosylated HIV-1_{MN} gp120 (Virostat, Portland) were added to individual wells of 96-well Immulon plates (Dynex technologies Inc, Virginia, USA). After incubating 16-20 hr at 4°C, the plates were washed three times with washing buffer (Kirkegaard and Perry Laboratories, Gaithersburg, Maryland) and 200 µl of blocking buffer (Kirkegaard and Perry Laboratories, Gaithersburg, Maryland, USA) was added and the plates were incubated for 1 hr at 24°C. After the blocking was complete, duplicated sets of each serum sample were diluted serially in 3-fold increments (Starting at 1:10) in blocking buffer and incubated for 1 hr at room temperature. Then, the plates were washed six times with washing buffer and 100 µl of horseradish peroxidase-labelled goat anti-mouse IgG (Sigma Immunochemicals, USA), diluted in 1/2000 in blocking buffer, was added to each well and the plates were incubated for 1 hr at 24°C. The plates were washed an additional six times with washing buffer and 100 µl of ABTS substrate (Kirkegaard and Perry Laboratories, Gaithersburg, Maryland, USA) was added and the plates were incubated for 30 min at 24°C. The absorbance was measured at 405 nm using a Wallac dynamic reader model 1420. A similar procedure was conducted to measure gp120-specific IgG subtypes, IgG1, IgG2a and IgG2b, except that rat anti-mouse IgG1, IgG2a, and IgG2b antibodies conjugated to horseradish peroxidase (diluted 1:8000, 1:2000, 1:1000 and 1:1000, respectively; BioSource International, Keystone, USA) were used in place of the goat anti-mouse IgG..

Enzyme-linked immune spot (ELISPOT) assay for Interferon-γ-secreting cells: Four weeks after the final vaccination spleens were aseptically removed from the vaccinated mice and single cell suspensions were prepared by teasing whole spleens between two sterile glass microscope slides. The erythrocytes were lysed in Tris-buffered ammonium chloride pH 7.4 (Biofluids); the unlysed cells were harvested by centrifugation at 250 x g for 10 min and washed two times in RPMI-1640 medium containing 2% (v/v) fetal bovine serum (FBS; Hyclone). Interferon-γ (IFN-γ-secreting cells (ISCs) specific for gp120 were enumerated using an ELISPOT assay. Briefly, 96-well filtration plates (Millipore, Bedford, MA) were coated overnight with 100 µl of rat anti-mouse IFN-γ antibody (clone R4-6A2, Pharminogen, San Diego, CA) and 5 µg/ml of PBS. After 24 hours, the plates were washed three times with washing buffer (PBS containing 0.5 % (v/v) Tween-20; Sigma). The plates were blocked with 200 µl of complete RPMI-1640 containing 10% (v/v) fetal bovine serum (FBS) at 37°C. After one hour, the plates were washed three times with washing buffer. Three-fold dilutions of immune spleen cells in complete RPMI-1640 containing 10% (v/v) FBS were added to the wells along with 20 units of recombinant mouse interleukin-2 (IL-2; R&D) and 10⁵ 10-ME fibroblasts per well, with or without the peptide P18_{MN} (i.e., NH₂-RIHIGPGRAFYTTKN-COOH) (GenBank accession # NM 001101). After culturing the cells for 24-36 hrs at 37°C in a humidified 5% CO₂ atmosphere, the plates were washed 3 times with H₂O and three times with washing buffer, and then incubated for two hours at 25°C with 100 µl of PBS containing 0.5% (v/v) Tween-20, 1% (w/v) BSA (Sigma) and biotinylated anti-mouse IFN-γ antibody clone XMG1.2 (2 µg/ml; Pharminogen, San Diego, CA). Subsequently, the plates were washed 3 times with washing buffer and then 100 µl of ExtraAvidin-alkaline phosphatase (Sigma, St. Louis MO) diluted 2000-fold in washing buffer was added to each well. After an additional incubation period of 30 min at 25°C, the plates were washed a further 3 times with washing buffer and developed using freshly prepared BCIP/NBT substrate (Sigma).

CD8⁺ and CD4⁺ cells were depleted by negative selection from splenocytes that had been stimulated for 6 days *in vitro* with the V3 peptide using Dynabeads according to the manufacturer's protocols (Dynal). Briefly, 100 µl of mouse anti-CD8 Dynabeads or mouse anti-CD4 Dynabeads were washed three times in HBSS and then resuspended in 100 µl of complete RPMI medium. The Dynabeads were then added to tubes containing 10⁷ splenocytes suspending 2 ml complete RPMI medium and incubated for 20 minutes at 4°C, with continuous gentle agitation. The spleen cell suspensions were placed into a Dynal magnet for 3 minutes and the non-attached cells were aspirated (CD8-depleted or CD4-depleted cells) and used in the above ELISPOT assay or ⁵¹Cr-release assay.

Inhibition of MHC Class-1 presentation was accomplished by adding an H-2k^{d}-specific mAb during the final stimulation step in the ELISPOT assay described above. The purified anti-mouse class I mAb (anti-H-2k^{d} clone SF1-1.1; Pharmingen) was dialysed in PBS overnight to remove the azide. This antibody or dialyzed isotype control antibody (mouse IgG2a; Pharmingen), were added to the splenocytes at a final concentration of 5 µg/ml, along with V3 peptide (5 µg/ml).

Quantitation of cytokines in culture supernatants: Cytokines were quantitated using commercially available capture ELISA kits (e.g., The IFN-γ ELISA kit, Cat. No. EM-1001-1 from Endogen, Woburn, MA; the interleukin-4 (herein called "IL-4") ELISA kit from R&D Systems, Minneapolis, MN, Cat. No. M4000; the interleukin-5 (herein called "IL-5") ELISA kit cat # M5000; the interleukin-10 (herein called "IL-10") ELISA kit Cat. No. M1000, from R&D Systems, Minneapolis, MN) or the U-Quant Facility (Institute of Human Virology, University of Maryland Biotechnology Institute, Baltimore, MD), that uses the aforementioned capture ELISA kits to measure IFN-γ, IL-4, IL-5, and IL-10 levels.

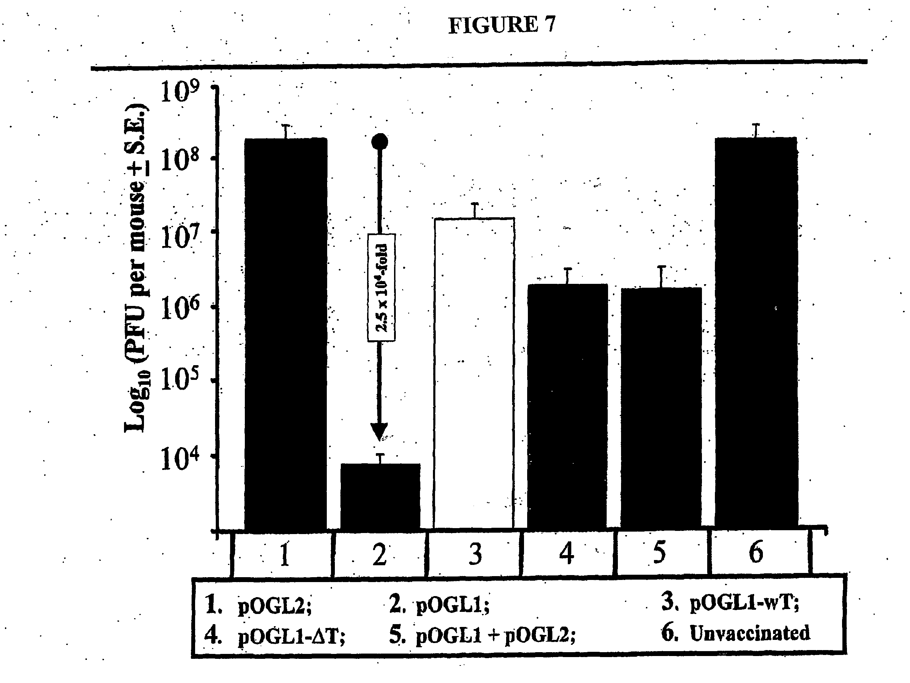

The results of the serum IgG response to HIV-1 gp120 measured in sera are shown in Figure 5 and Table 2. The mice vaccinated with pOGL1 developed a strong serum IgG response against gp120, whereas the mice vaccinated with pOGL1-wT did not develop a strong anti-gp120 IgG response. The mice vaccinated with pOGL1 developed a strong serum IgG response against gp120, whereas the peak anti-gp120 IgG response that ensued following vaccination with pOGL1-wT was about 30-fold less than the response in pOGL1-vaccinated mice (Table 2). The kinetics of the gp120-specific serum IgG response in mice vaccinated with pOGL1-wT also differed from the analogous response in mice vaccinated with pOGL1. Thus, detectable gp120-specific IgG arose after a single dose of pOGL1, whereas this response was only marginally evident in mice vaccinated with 2 doses of pOGL1-wT (i.e., a titer of 1:30, 34 days after vaccination, compared to a titer of 1:1000 at the same time point in mice vaccinated with pOGL1; Figure 7).

**TABLE 2**

| **Vaccine** | **Relevant Genotype** | **BALB/C (Peak end-point Titer x⁻¹⁰)** | **C57BL/6 (Peak end-point titer x 10⁻¹)** |
|---|---|---|---|
| **pOGL2** | *htat* | <30 | <30 |
| **pOGL1** | *hgp120* | 10,000 | 30,000 |
| **pOGL1-wT** | *hgp120* + *htat* | 300 | 1,000 |

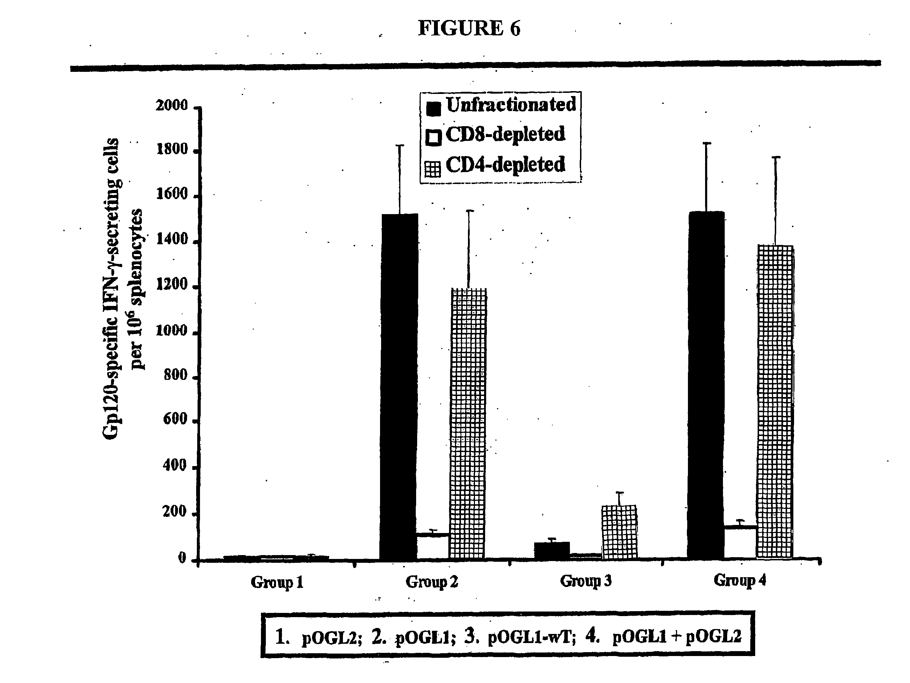

To assess the magnitude of the CD8⁺ T cell response in the vaccinated mice, splenocytes were harvested 28 days after the fourth vaccination from half (3 mice) of the mice vaccinated with pOGL1, pOGL1-wT or pOGL2 as described above. Subsequently, unfractionated, CD4⁺ T cell- and CD8⁺ T cell-depleted splenocytes were used to enumerate gp120-specific, interferon-γ (IFN-γ)-secreting CD8⁺ T cells by ELISPOT, as described above. The results of the ELISPOT assay as shown in Figure 6 indicate that mice vaccinated with plasmid pOGL1 develop a strong gp120-specific IFN-γ-secreting CD8⁺ T cell response, whereas the mice vaccinated with pOGL1-wT did not elicit a measurable gp120-specific IFN-γ-secreting CD8⁺ T cell response. Taken together, the serum IgG and CD8⁺ T cell data show that co-expression of immunoregulatory protein Tat with vaccine antigen gp120 by CED vaccine pOGL1-wT results in a complete block in the gp120-specific serum IgG and splenic CD8⁺ T cell responses that develop when mice are vaccinated with pOGL1, which expresses gp120 alone.

In a parallel experiment, mice were vaccinated with four (4) 100 µg doses of pOGL2 or pOGL1-wT using the above intervals between doses. The mice were then boosted with mice 10 µg of purified glycosylated gp120 formulated in 100 µl of Freud's complete adjuvant FCA 92 days after the first vaccination. ELISAs were used to monitor these mice for the development of gp 120-specific serum IgG in sera collected at weekly intervals, as above. The results of the ELISAs showed that both groups (e.g., pOGL2 and pOGL1-wT-primed) developed a high-level gp120-specific serum IgG response. However, the response in the pOGL2 primed mice was predominated by a strong gp120-specific IgG1 and IgG2a subtype responses, whereas in the pOGL1-wT-primed mice the serum IgG response gp120-specific produced gp120 specific IgG1 and a small IgG2a response as shown in Table 3.

**TABLE 3**

| Priming vaccine | gp120-specific IgG1 (End-point titer X 10⁻¹) | gp120-specific IgG2a (End-point titer X 10⁻¹) | IgG1:IgG2a ratio |
|---|---|---|---|
| None | 30,000 | 10,000 | 3 |
| POGL2 | 30,000 | 10,000 | 3 |
| POGL1-wT | 30,000 | 300 | 100 |

This observation provides evidence that the lack of gp120-specific serum IgG and CD8⁺ T cell responses during the initial vaccination schedule with the CED vaccine pOGL1-wT was the result of systemic tolerance and mediated through immune deviation. An analogous form of systemic tolerance through immune deviation is observed in mice vaccinated in the anterior chamber of the eye (Koevary and Beandry. Ocul. Immunol. Inflamm., 8: 39-47 (2000). Immune deviation is a powerful tool for inducing or restoring tolerance and is useful in the prevention and treatment of inflammatory diseases, such as host-versus-graft tissue rejection and autoimmune diseases.

To determine whether co-expression of Tat and gp120 on a CED vaccine were necessary to yield the full immunoregulatory effect of Tat, a second experiment was conducted in which groups of 6 BALB/c mice were vaccinated intramuscularly with 40 µg of either pOGL2, pOGL1, pOGL1-wT or a mixture of 40 µg each of pOGL1 and pOGL2 (the latter expresses Tat alone). The mice were given booster vaccinations comprised of the same dose of vaccine 14 and 42 days after the primary vaccination. In a similar vein to the first experiment, mice vaccinated with pOGL1 developed a strong gp120-specific serum IgG response, whereas the mice vaccinated with pOGL1-wT developed only low-level gp120-specific serum IgG. In contrast, the mice vaccinated with the mixture of pOGL1 and pOGL2 developed a gp120-specific serum IgG response that was similar in magnitude to the response induced by pOGL1 as shown in Figure 6. Importantly, this result shows that the immunoregulatory properties of Tat are better harnessed in the context of the CED vaccine than in the context of the DNA vaccine mixture.

In summary, the experiments described in this example show that a CED vaccine (E.g., pOGL1-wT) that expresses an antigen (E.g., HIV-1 gp120) and an immunoregulatory protein (E.g., HIV-1 Tat) induces systemic tolerance against the antigen through immune deviation. Thus, CED vaccines comprised of an antigen and Tat, such as pOGL1-wT, are useful for inducing or restoring systemic tolerance through immune deviation; hence CED vaccines containing Tat are useful for the prevention and treatment of inflammatory diseases (E.g., Host-versus-graft tissue rejection and autoimmune diseases).

In addition, we have shown that CED vaccine pOGL1-wT exploits the immunoregulatory properties of Tat more effectively than a mixture of two conventional DNA vaccines (E.g., pOGL1 + pOGL2). Thus, CED vaccines are an enhanced modality for utilizing immunoregulatory agents (E.g., Growth factors, hormones, cytokines and chemokines).

### Example 4

### Recall immunity reveals additional evidence of Tat-mediated immune deviation

Construction of a mutant derivative of Tat: A mutant derivative of Tat (hereinafter referred to as Δ*htat*) was constructed to assess the role of the LTR-activating property of Tat in the modulation of the host response. Δ*htat*, which lacks a 66 bp region encoding amino acids 30 to 51 , was derived from the wild type *htat* gene (i.e. pOGL2) by two rounds of PCR. During the first round of PCR two fragments were generated: one fragment (135 bp) encoding the 5-prime end of Δ*htat,* which was amplified using the *htat* forward primer shown in Example 1, with reverse primer 5'-GCCAAATACATGGCCATTGAGCCCGTGGACCCTCGCCTGGAGCCCT (SEQ ID NO: 19); and a second fragment (208 bp) encoding the 3-prime end of Δ*htat,* which was amplified using the *htat* reverse primer shown in example 1 with forward primer 5'- ATAAGAATCTCGAGCAGCTGGAATTCGCGGCCGGCTGATCAG (SEQ ID NO: 20). The two PCR-generated fragments encoding the 5' and 3' ends of Δ*htat* were fractionated by agarose gel electrophoresis and purified using the Qiagen gel fragment purification Kit (Qiagen, CA; Cat No. 28704). A second PCR reaction was then executed comprised of the purified fragments encoding the 5' and 3' ends of Δ*htat* as template and the original *htat* primers. The latter PCR generated the complete Δ*htat*-encoding sequence (276 bp) flanked by *MscI* and *XhoI* recognition sites. The PCR-generated fragment was fractionated by agarose gel electrophoresis and purified using the Qiagen gel fragments purification Kit (Qiagen, CA; Cat No. 28704) and digested with *MscI* and *Xho*I. Simultaneously, pOGL1-wT was digested with *Bst*EII and *Xho*I, resulting in two fragments, a 5051 bp fragment encoding pcDNA3.1_{ZEO} and a 2271 fragment encoding the *hgp120::*CITE*::htat* dicistronic gene cassette. The *hgp120*::*CITE*::*htat* fragment was fractionated by agarose gel electrophoresis, purified using the Qiagen gel fragment purification Kit (Qiagen, CA; Cat No. 28704) and digested with *Msc*I. The resultant 1939 bp fragment encoding *hgp*120::CITE was fractionated by agarose gel electrophoresis, purified using the Qiagen gel fragment purification Kit (Qiagen, CA; Cat No. 28704). A three way ligation reaction was then conducted using the purified *BstEII-XhoI* pcDNA3.I_{ZEO}, *BstEII-MscI hgp120mn::CITE,* and *MscI-XhoI Δhtat* fragments as substrates for the ligation reaction. The annealed DNA was introduced into *E*. *coli* strain Stable2 by electroporation and individual clones were screened by analyzing small-scale plasmid DNA preparations isolated from individual clones for the appropriate *Bst*EII, *Xho*I, *MscI* digestion patterns. An isolate (referred to herein as strain "T145") was identified that contained the anticipated plasmid (referred to herein as "pOGL1-ΔT") and was stored at -80°C. Additional analysis by restriction endonuclease digestion, PCR of pOGL1-ΔT DNA, and dideoxynucleotide sequencing of *Δhtat* in pOGL1-*Δ*T was conducted to verify that the PCR-generated fragment was not altered during construction.

Cell line culture and transfection procedures: Murine P815 (H-2d; ATCC No. TIB-64) cells were obtained from the American Type Culture Collection (Manassas VA). HeLa-(LTR-*lac*Z) cells were obtained from the National Institutes of Health AIDS Research and Reference Program (NIAID, Bethesda MD). The murine and human cell lines were maintained in complete medium (CM), which was composed of RPMI 1640 medium (Life Technologies, Gaithersburg MD) supplemented with 10 mM HEPES pH 7.3 (Life Technologies, Gaithersburg MD), 10% (v/v) fetal calf serum (Gemini Bioproducts, Calabasas, CA), 4 mM glutamine (Life Technologies, Gaithersburg MD, 1 mM sodium pyruvate (Life Technologies, Gaithersburg MD), 100 µg/ml each of penicillin and streptomycin (Life Technologies, Gaithersburg MD). Plasmids pOGL1, pOGL2, pOGL1-wT and pOGL1-AT were introduced into P815 cells using the FuGENE^{®} multi-component transfection system (Roche Molecular Biochemicals, Indianapolis, IN cat # 1-815-091). Stable cell lines were selected 7 days after the transfection by adding zeomycin (200 ul/ml) to the culture media. Expression of β-galactosidase by HeLa-(LTR-lacZ) cells was determined using a fluorochromogenic β-galactosidase assay system (Promega, Madison City, WI; Cat. No. E2000); fluorochrome levels were measured using a Wallac Victor-2D fluorometer (Turku, Finland). β-galactosidase activities were expressed in terms of relative light units (RLUs) per mg of protein per hour.

Vaccination of mice: Female specific-pathogen free BALB/c mice aged 6-8 weeks were obtained from Charles River (Bar Harbor, Maine). The mice were maintained in a microisolator environment and allowed to feed and drink *ad lib.* To evaluate the immunogenicity of the DNA vaccines developed for this study, groups of mice were vaccinated intramuscularly with 40 µg of endotoxin-free (<5 EU per mg of DNA) plasmid DNA suspended in 0.85% (w/v) NaCl (Sigma, St. Louis, MO), as described in Wolff, et al., Science, 247, 1465-1468 (1990). Booster vaccinations were injected using the same vaccination protocol 14 and 42 days after primary vaccination.

Measurement of serum IgG specific to Tat: Venous blood (ca. 100 µl per mouse) was collected before and at regular intervals after vaccination, and allowed to coagulate; after centrifugation the sera were collected and stored at -20°C. A standard enzyme-linked immunosorbent assays (ELISA) was used to measure the level of Tat-specific IgG in triplicate sets of sera serially diluted in 3-fold increments (Starting at 1:30). Prior to adding the sera, individual wells of 96-well Immulon plates (Dynex Technologies Inc, Virginia, USA) were coated with purified rTat protein (Advanced BioScience Laboratories, Kensington, MD). Alkaline phosphatase-labelled goat anti-mouse IgG (Sigma, St. Louis MO) was used as secondary antibody and absorbance values were measured at 450 nm. End-point titers were calculated by interpolation, using the mean background absorbance plus three standard deviations as the cut-off.

Measurement of cell-medicated immune responses: Gp120-specific T cell proliferation was measured using splenocytes that were harvested 10 days after the final vaccination and placed in CM containing β-mecaptoethanol (5 µM). T cell proliferation was measured by tritiated thymidine (³H-TdR) incorporation, as described in Wu, et al., AIDS Res. Hum. Retrovir. 13, 1187-1194 (1997); the stimulants used in each proliferation assay included fully-glycosylated purified HIV-1_{MN} gp120 (10 µg/ml; Virostat Inc., Portland OR), a mitogen control (anti-mouse CD3 (Pharmingen, San Diego, CA)), and endotoxin-free ovalbumin (10 µg/ml; Sigma, St. Louis MO) as a negative control.

Single cell suspensions of splenocytes (Wu, et al., AIDS Res. Retrovir., 13:1187-1194 (1997)) were prepared 10 days after the final vaccination. The splenocytes were cultured for 6 days in CM containing 5 µg/ml of the immunodominant peptide of HIV-1 Env (Takahashi, et al., Proc. Natl. Acad. Sci., 85, 3105-3109 (1988)), designated P18_{MN} (RIHIGPGRAFYTTKN). 10 IU recombinant mouse interleukin-2 (R&D Systems, Minneapolis, MN) was added to these cultures after the initial three days of *in vitro* culture. Following *in vitro* stimulation, Env-specific IFN-γ-secreting T cells were enumerated using an IFN-γ-ELISPOT assay, as originally described by Versteegen, et al., J. Immunol. Meth., 111, 25-29 (1988) and subsequently modified (Miyahira, et al., J. Immunol. Meth., 181, 45-54 (1995)). Three-fold dilutions of the splenic cell populations. (from 10⁵ to 10³ cells) were mixed with 10⁵ P815 cells in complete medium containing 10 IU recombinant mouse interleukin-2 (R&D Systems, Minneapolis, MN), either with or without 5 µg/ml of peptide P 18_{MN}.

CD4⁺ or CD8⁺ cells were depleted by negative selection from splenic cells using CD4⁺- or CD8⁺-specific Dynabeads^{R}, respectively, according to the manufacturer's protocols (Dynal, Lake Success, NY). The unfractionated, and CD4- and CD8-depleted cells were used immediately in the IFN-γ-ELISPOT assays.

Vaccinia-Env challenge: The level of antiviral protection induced by each DNA vaccine modality was determined using a vaccinia-env challenge model, as described in Belyakov, et al., Proc. Natl. Acad. Sci., 95:1709-1714 (1998). Briefly, inocula of vP1174 were prepared by culturing the recombinant vaccinia on BSC-1 cells until 90% of the cells were lysed. The lysed cells were removed from the culture supernatants by centrifugation at 4,000 x g for 10 min and aliquots of the supernatants were stored in liquid nitrogen until used. The culture supernatants typically yielded about 5 x 109 vP1174 pfu/ml, as determined by a direct plaque assay on BSC-1 cells. Mice were inoculated with 3 x 107 pfu of vP 1174 via intraperitoneal injection six to nine days after vaccination. Six days after the challenge the ovaries of the mice were harvested and homogenized with a mechanical tissue grinder. The homogenates were clarified by centrifugation at 4000 x g for 10 min and the vP 1174 pfu in the resultant supernatants were enumerated by infecting BSC-1 cell monolayers with 10-fold serial dilutions of these fluids and counting plaques after two days in culture at 37°C in a 5% CO2 environment.

Expression of gp120 and Tat: Experiments were conducted to verify that the DNA vaccines used in this study expressed comparable levels of gp120 and/or biologically active Tat. To determine whether expression of Tat influenced the level of gp120 expression, a semi-quantitative gp120 capture assay (Abacioglu, et al., AIDS Res. Hum. Retrovir. 10: 371-381 (1994)) was employed to measure the level of gp120 expressed by P815 cells transiently transfected with pOGL1-wT, pOGL1-ΔT, or parent plasmid pOGL1 (Table 4). The results demonstrate that gp120 is expressed at comparable levels by pOGL1, pOGL1-wT and pOGL1-ΔT, which adds further support to the notion that co-expression of Tat did not prevent gp120 expression in *vivo.*

The activity of the Tat protein expressed by plasmids pOGL2, pOGL1-wT and pOGL1-ΔT was assessed using HeLa-LTR-*lac*Z cells transiently transfected with these plasmids, as outlined in the methods. These assays demonstrated that plasmids pOGL2 and pOGL1-wT activated significant levels of LTR-*lacZ* expression, whereas plasmid pOGL1-ΔT, which harbors the deletion-modified *tat* gene, *Δhtat,* did not activate measurable levels of LTR*-lacZ* expression as shown below in Table 4.

**TABLE 4**

| **Vaccine** | **Expression cassette** | **gp120 expression (pg/10⁶** P815 **cells)** | **LTR-*lac*Z (RLU x 10⁵)** |
|---|---|---|---|
| pOGL1 | P_{CMV}-*hgp120_{MN}* | 278 ± 55 | Not detected. |
| pOGL2 | P_{CMV}*-htat_{MN}* | <10 | 2.78 + 0.7 |
| pOGL1-wT | P_{CMV}*-hgp120*-IRES*-htat* | 356 ± 63 | 0.97±0.3 |
| pOGL1-*Δ*T | P_{CMV}*-hgp120-*IRES*-Δhtat* | 198 ± 34 | Not detected. |

Influence of Tat on the induction of gp120-specific cell-mediated responses: To assess the CD4⁺ T cell responses following vaccination with pOGL1, pOGL1-wT, or pOGL1-*Δ*T, BALB/c mice were vaccinated using the above protocol and the level of gp 120-specific T cell proliferation was measured ³H-TdR incorporation in splenocytes 10 days after the third vaccination. In contrast to the humoral responses, the proliferation assays revealed little difference in the magnitudes of the gp120-specific proliferation following three doses of the gp120-expressing DNA vaccines. The results suggest that Tat does not significantly alter the level of MHC class II-restricted presentation to, and CD4⁺ T cell recognition of, gp120 in mice.

Tat RNA vaccines induce low numbers of TFN-γ-ELISPOT: In parallel, the Tat-specific IFN-γ-ELISPOT responses were evaluated in mice vaccinated intramuscularly three times with 40 µg of pOGL2 or pOGL1-wT, using the same spacing between the doses as before. Negative control mice were vaccinated with pOGL1. Enumeration of IFN-γ-ELISPOTs to full-length Tat or a peptide spanning Tat amino acids 31-50 revealed relatively low numbers of spot-forming cells (i.e. *ca.* 150-200 Tat-specific IFN-γ-ELISPOTs per 10⁶ splenocytes) in mice vaccinated with either pOGL2 or pOGL1-wT (data not shown).

Tat diminishes antiviral immunity to gp120: Antiviral immunity in the vaccinated mice was assessed 28 days after the third vaccination using the vaccinia-env challenge model described by Belyakov, et al., *supra.* The vaccinated mice were challenged with 3x10⁷ pfu of vaccinia-*env* vector strain vT26, which expresses Env of HIV-1_{MN}, as described in Belyakov, *supra.* Six days after the challenge the ovaries of the mice were harvested and the number of infectious challenge vT26 viral particles present in these tissue samples was enumerated according to Belyakov, *supra.* The results of the challenge assay (Figure 7) showed that only vaccination with pOGL1 afforded strong antiviral immunity against vaccinia-*env*, whereas the mice vaccinated with pOGL1-wT, pOGL1-*Δ*T or a mixture of pOGL1 and pOGL2 did not develop significant antiviral immunity against the challenge virus.

A Tat submit vaccine confers protection against the immune modulating activity of Tat: To assess whether vaccination with a Tat subunit vaccine afforded protection against the immune-modulating properties of Tat, a group of 3 BALB/c mice was vaccinated with three 100 µg doses of purified recombinant Tat (rTat; Advanced BioScience Laboratories, Kensington, MD) on days 0, 14 and 28 mixed with 100 µg of LPS from *Escherichia coli* strain MLK986, which produces a non-pyrogenic lipid A (Hone, et al., J. Hum Virol., 1:251-256 (1998)) and posses similar adjuvant activity to that of monophosphoryl-lipid A (MPLA). To assess the immunogenicity of a candidate Tat DNA vaccine, a second group of BALB/c mice was vaccinated with three 100 µg doses of pOGL2 using that same spacing between doses as used with the rTat vaccine. Control groups of mice remained unvaccinated or were vaccinated with 3 100 µg-doses of pcDNA3.1_{NEO} intramuscularly, as above.

Analysis of the serum IgG responses to Tat in the mice vaccinated with rTat revealed a significant IgG response to Tat (Reciprocal end-point titer of *ca.* 50,000), whereas the serum IgG antibodies to Tat were not significantly elevated in the mice vaccinated with pOGL2 (Reciprocal end-point titer < 100).

To assess the protective properties of the two Tat vaccine modalities, the unvaccinated control mice and the mice pre-vaccinated with rTat or pOGL2 were given a second series of vaccinations comprised of three 100-µg doses of pOGL1-wT 2, 4 and 8 weeks after the final vaccination with rTat and pOGL2. The additional control mice that were pre-vaccinated with pcDNA3.1_{NEO} were given a second series of vaccinations comprised of three 100-µg doses of pOGL1, 2, 4 and 8 weeks after the final vaccination with pcDNA3.1_{NEO}. Nine days after the second series of vaccinations were completed, each group of mice was challenged with 3 x 10⁷ pfu of vaccinia-*env* vector strain vT26. The levels of antiviral immunity in each group of mice were determined by harvesting the ovaries of the mice six days after the challenge and enumerating the numbers of infectious vT26 viral particles present in these tissue samples (see methods above). This challenge assay showed that prior vaccination with oxidized-rTat afforded strong protection against the immune modulating activity of Tat, whereas the mice vaccinated with pOGL2 did not develop significant levels of protection against the immune modulating activity of Tat (data not shown).

Importantly, an appropriately formulated Tat vaccine was capable of blocking Tat-mediated immune modulation. This is the first report showing that immunization against Tat is capable of completely blocking Tat-mediated immune modulation in an animal model.

### Example 5

### The LTR-activating activity of Tat is not required for the induction of immune deviation

To investigate the immune modulating properties of the ΔTat on plasmid pOGL1-AT, a similar mouse immunogenicity assay to the one described in Example 4 was conducted using of 5 groups of 6 BALB/c mice vaccinated intramuscularly with two 40 µg-dosses of endotoxin-free plasmid DNA at a 14 day interval, as shown in the Table 5. A third 40 µg-dose was injected intramuscularly 28 days after the second dose.

**TABLE 5**

| **Group** | **Vaccine** | **Relevant genotype** |
|---|---|---|
| 1 | pOGL2 | *htat*⁺ |
| 2 | pOGL1 | *hgp*120⁺ |
| 3 | pOGL1-wT | *hgp*120⁺ and *htat*⁺ (Dicistronic) |
| 4 | pOGL1-ΔT | *hgp120⁺* and *Δhtat*⁺ (Dicistronic) |
| 5 | pOGL1+pOGL2 | *hgp120*⁺ and *htat⁺* |

The serum IgG responses to HIV-1 gp120 were measured using pooled sera collected at weekly intervals from the above groups of mice. Mice in group 2 (e.g. vaccinated with pOGL1 alone), group 4 (e.g. vaccinated with pOGL1-ΔT) and group 5 (e.g. vaccinated with a mixture of pOGL1 and pOGL2) developed a serum IgG responses against gp120, whereas the mice in group 3 (vaccinated with the dicistronic DNA vaccine pOGL1-wT) only developed a meager gp120-specific serum IgG response and the negative control mice in group 1 (e.g. vaccinated with pOGL2 alone) did not develop an anti-gp120 IgG response, as shown in Figure 5.

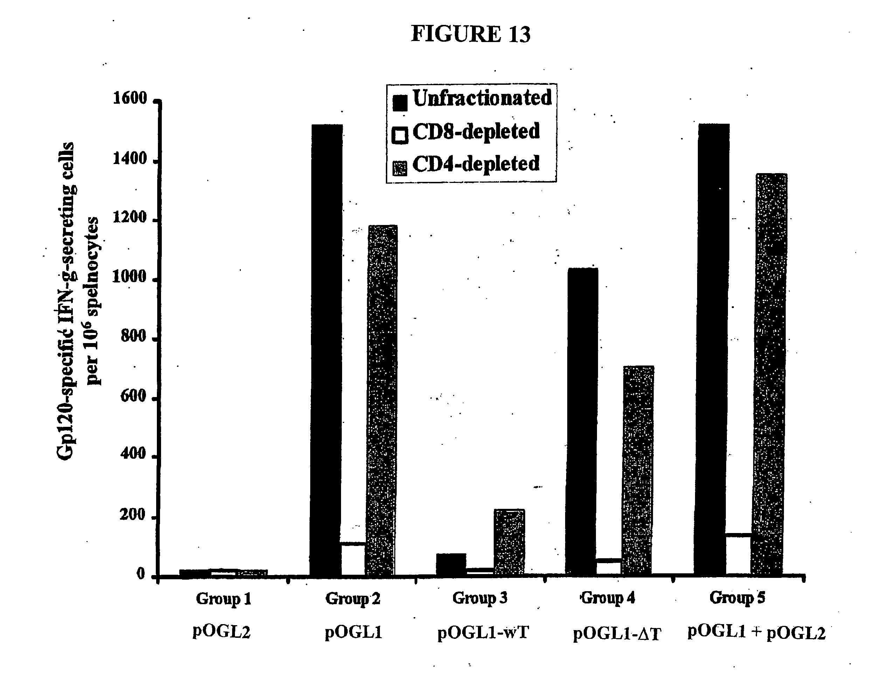

Using an IFN-γ-specific ELISPOT assay, Env-specific CD8⁺ T cell responses were enumerated in unfractionated, CD4⁺ T cell- and CD8⁺ T cell-depleted splenocytes harvested from half of the vaccinated mice 28 days after the final vaccination. The results of the ELISPOT assay (Table 6) show that pOGL1 (Group 2), pOGL1-ΔT (Group 4) and the mixture of pOGL1 and pOGL2 (Group 5) elicited gp120-specific IFN-γ-secreting CD8⁺ T cell responses. In contrast, pOGL2 (Group 1) and pOGL1-wT (group 3) did not elicit measurable gp120-specific IFN-γ-secreting CD8⁺ T cell responses. (See Figure 13)

**TABLE 6**

| **Group** | **Vaccine** | **Unfractionated gp120-specific ISCs per 10⁶ splenocyte.** | **CD8⁺ T cell- depleted gp120-specific ISCS per 10⁶ splenocyte.** | **CD4⁺ T cell- depleted gp120-specific ISCs per 10⁶ splenocyte.** |
|---|---|---|---|---|
| 1 | pOGL2 | <20 | <20 | <20 |
| 2 | pOGL1 | 1519 | 110 | 1178 |
| 3 | pOGL1-wT | 74 | <20 | 220 |
| 4 | pOGL1-ΔT | 1030 | 48 | 703 |
| 5 | pOGL1 + pOGL2 | 1518 | 135 | 1352 |

As before, the remaining three mice in each group were challenge with 10⁸ pfu of vaccinia-Env vector strain vT26 (AIDS Repository, National Institute of Allergy and Infectious Disease, Bethesda MD), which expresses Env of HIV-1_{MN}, as described (Belyakov, et al., *supra* (1998)). Five days after the challenge the ovaries of the mice were harvested and the number of infectious challenge virus particles present in these tissues samples was enumerated (Belyakov, et al., *supra* (1998)). The results of the challenge assay showed that only vaccination with pOGL1 afforded strong anti-viral immunity against vaccinia-Env; the mice other groups, including the mice vaccinated with pOGL1-ΔT, did not develop significant antiviral immunity.

The serum IgG and CD8⁺ T cell ELISPOT data showed that pOGL1-ΔT does not suppress the Env-specific response to the same extent as pOGL1-wT. However, the viral challenge data show that mutant Tat, ΔTat, is still capable of mediating immune deviation, since mice vaccinated with pOGL1-ΔT did not develop measurable anti-viral immunity. Thus, the LTR-activating activity of Tat is not required for the induction of immune deviation.

### Example 6

### Construction of a co-expression DNA vaccine encoding a vaccine antigen and an immunostimulatory protein

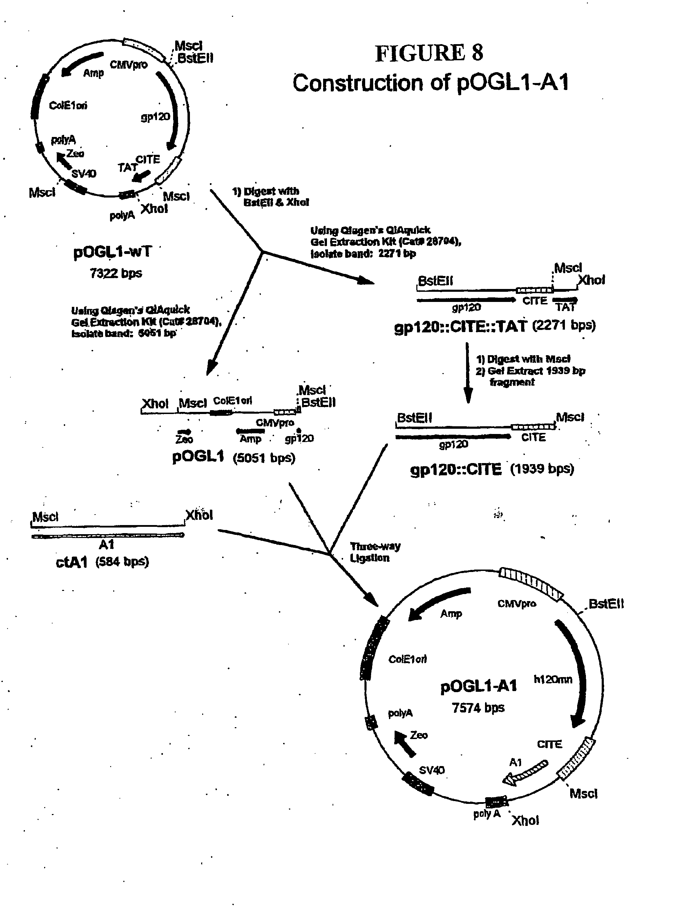

DNA vaccine construction strategy: A novel DNA vaccine that co-expresses an antigen (e.g., gp120) and an adjuvant (e.g., CtxA1), referred to herein as "pOGL1-A1", was constructed by replacing *tat* in pOGL1-wT with sequences encoding the A1 domain of CT (i.e. CtxA1). The strategy used to construct the plasmid pOGL1-A1 is shown in Figure 8. All procedures used in the construction of pOGL1-A1, including restriction endonuclease digestions, agarose gel electrophoresis, DNA purification, DNA ligation and PCR are generally described hereinabove.

Specifically, the recombinant plasmid pOGL1-A1 was constructed, wherein gp120 of HIV-1_{MN} and the A1 domain of the A subunit of cholera toxin (referred to herein as "Ctx") are co-expressed. Ctx is a well-known mucosal adjuvant (Xu-Amano, et al., J. Exp. Med., 178:1309 (1993); VanCott, et al., Vaccine, 14:392 (1996); Jackson, R. J. et al., Infect. Immun., 61:4272 (1993); Marinaro, M. et al., Ann. New York Acad. Sci., 795:361 (1996); Yamamoto, S. et al., J. Exp. Med., 185:1203 (1997); Porgador, et al., J. Immunol., 158:834 (1997); Lycke and Holmgren, Monogr., Allergy, 24:274 (1988); Hornquist and Lycke, Eur. J. Immunol., 23:2136 (1993); Homquist, et al., Immunol., 87:220 (1996); Agren, et al., Immunol. Cell Biol., 76:280 (1998)). The adjuvant activity of Ctx is mediated by the A1 domain of the A subunit of Ctx (herein referred to as CtxA1). Chimeric proteins comprised of an antigen fused to CtxA1 demonstrate the CtxA1 is an adjuvant (Agren, et al., J. Immunol., 164:6276 (2000); Agren, et al., Immunol. Cell Biol., 76:280 (1998); Agren, et al., J. Immunol., 158:3936 (1997)). However, the utilization of the A1 domain in a CED vaccine has not heretofore been reported.

The plasmid vector of pOGL1-A1 is pcDNA3.1_{ZEO}, which was purchased from Invitrogen (Carlsbad, CA). DNA encoding the IRES of equine encephalitis virus, herein referred to as the cap-independent translational enhancer (U.S. patent number 4,937,190), was amplified from plasmid pCITE4a (Novagen, Madison WI; Cat. No. 69912-1; U.S. patent number 4,937,190) using forward primer 5'-ATAAGAATGCGGCCGCTAAGTAAGTAACTTAAGTTCCGGTTATTTTCCACG ATATTGCCGTCTTTTGGCAA (SEQ ID NO 16) and reverse primer 5'-GCCAAATACATGGCCATATTATCATCGTGTTTTTCAAAGGAA (SEQ ID NO: 17).

DNA encoding the gp 120 gene, which was optimized for expression in mammalian cells (Andre et al., *supra,* (1998); Haas et al., *supra,* (1996)), was amplified from a plasmid pEFla-syngp120_{MN} (Andre et al., *supra,* (1998); Haas et al., *supra,* (1996)) using forward primer 5'-GGGGGGGGATCCATGCCCATGGGGTCTCTGCAACCGCTG (SEQ ID NO: 14) and reverse primer 5'-GGGGGCGGCCGCTTATTAGGCGCGCTTCTCGCGC TGCACCACGCG. (SEQ ID NO: 15).

DNA encoding CtxA1 was amplified from plasmid pCVD002 provided by the Center for Vaccine Development, University of Maryland, Baltimore; Lochman and Kaper, J.

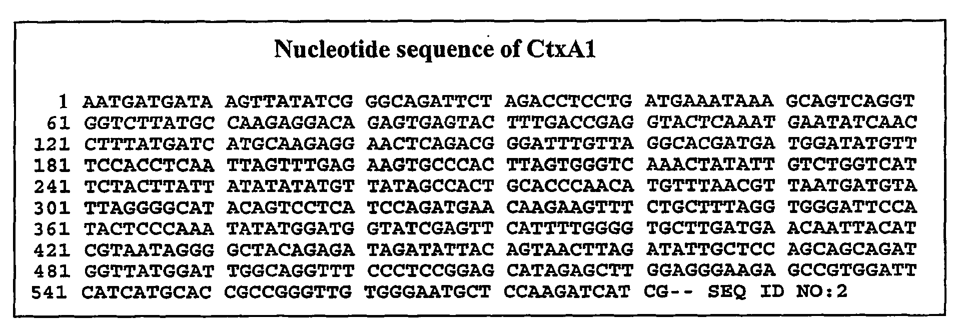

Biol. Chem., 258:13722 (1983)), which has a copy of cholera toxin genes *ctxA* and *ctxB.* The nucleotide sequence of *ctxA* was obtained from GenBank (Accession # A16422, SEQ ID NO: 1). CtxAl-encoding sequence (SEQ ID NO: 2) was amplified using forward primer 5'-CCC AAG CTT ATG AAT GAT GAT AAG TTA (SEQ ID NO: 3) and reverse primer 5'-GGG GCG GCC GCT TAC GAT GAT CTT GGA GC (SEQ ID NO: 4). The primers incorporated a *Hind*III recognition site to the 5'-end of the PCR-generated product and a *Not*I site to the 3'-end by primer extension. The PCR reaction mixture included 10 ul of both primers, 10mM dNTP, 50 ul of 10X PCR buffer without MgCl₂, 62.5 ul of 25 mM MgCl2, 2.5 ul of pRc/CMV (0.65*ug*/*u*l), 352.5 ul of DEPC treated water and 2.5 µl of AmpliTaq polymerase. The PCR protocol included 35 cycles of a temperature regime including 1 minute at 94°C, 2 minutes at 55°C and 3 minutes at 72°C.

The PCR-generated product was digested with *Hind*III and *Not*I and inserted into the pRc/CMV (Invitrogen, Carlsbad, CA), by ligation. The ligation reaction was terminated and the DNA was introduced into *E. coli* DHS_{α} by chemical transformation. The transformed bacilli were plated on LB plates supplemented with 100 µg/ml ampicillin at 37°C for 16 hr. Isolated colonies were selected and grown overnight in 3 ml of LB medium supplemented with 100 µg/ml ampicillin. DNA was extracted from overnight liquid cultures using a Qiagen mini plasmid DNA preparation kit (Cat No Q7106). Plasmid DNA was screened for insert by digesting with *Hind*III and *Not*1 followed by agarose gel electrophoresis. Several clones that tested positive for the *ctxA1* insert were also tested for *CtxA1* functional activity in 293 cells (ATCC # CRL-1573). Plasmid DNA was transfected into 293 cells using Superfect^{R} as per the manufacture's instructions (Qiagen, Valencia CA; Cat No 301305). Transfection efficiency was determined using a Promega β-Galactosidase assay as per the manufacture's instructions. cAMP production by transfected cells was assayed using a kit from Amersham as per the manufacture's instructions.

### Example 7

### Immunogenicity of a co-expression DNA vaccine encoding; a vaccine antigen and an immunostimulatory protein

The immunostimulatory activity of CED vaccine pOGL1-A1 was characterized by comparing the immunogenicity of DNA vaccine pOGL1 and pOGL1-A1 in BALB/c mice. Briefly, groups of 3 BALB/c mice were vaccinated intramuscularly with two 100 µg-doses of endotoxin-free plasmid DNA at 14 day intervals. Another group of 3 BALB/c mice received two 100 µg-doses of plasmid pcDNA3.1 DNA using the same protocol.

Sera were collected before or 10, 12, 22, 34, 50, 60 and 80 days after the first vaccination and used to measure the serum IgG response against HIV-1_{MN} gp120 by ELISA, as described above.

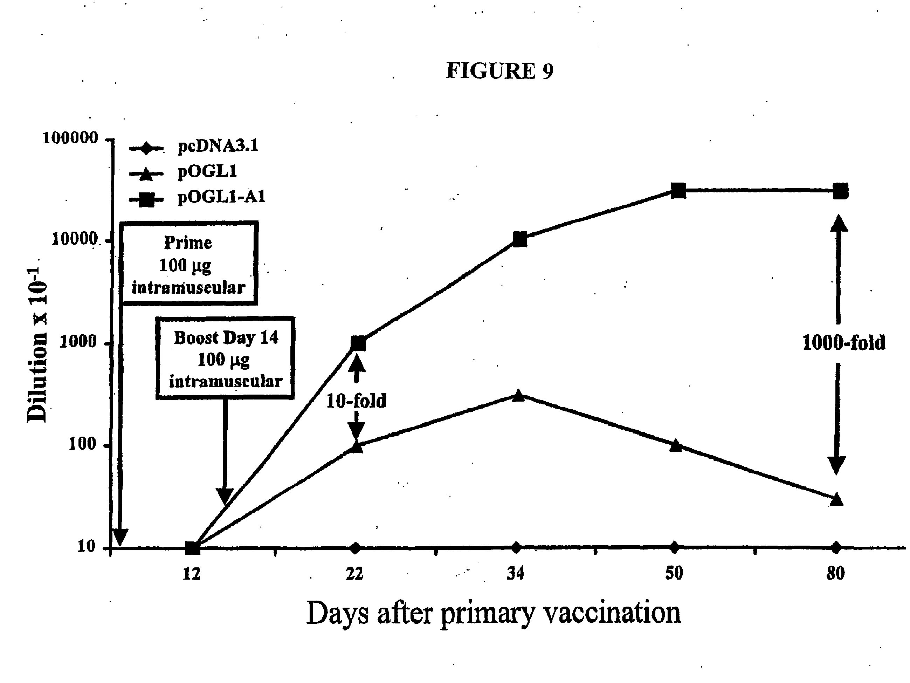

The results show that mice vaccinated with CED vaccine pOGL1-A1 develop a serum IgG response against gp120 that is 10-fold greater than the anti-gp120 serum IgG response in pOGL1-vaccinated mice 34 days after vaccination, when the peak response occurs in these latter mice. Following day 34, the serum IgG response continues to rise in mice vaccinated pOGL1-A1, whereas the response induced by pOGL1 wanes; 80 days after vaccination the gp120-specific response elicited by pOGL1-A1 is 1000-fold greater than the serum IgG response in mice vaccinated with pOGL1 or a mixture of pOGL1 and pcDNA-Al (Figure 9).

### Example 8

### A DNA vaccine that co-expresses an antigen and an adjuvant significantly augments the cell-mediated immune response to the antigen

To assess the magnitude of the CD8⁺ T cell response in the mice vaccinated with pOGL1-A1 and pOGL1, splenocytes were harvested 80 days after the first vaccination, as described above. Subsequently, un-fractionated splenocytes were used to enumerate gp120-specific, IFN-γ-secreting CD8⁺ T cells by ELISPOT, as described above. The results of the ELISPOT assay were similar to the results of the ELISA data, in that mice vaccinated with pOGL1-A1 developed a significantly stronger IFN-γ-secreting CD8⁺ T cell response than mice vaccinated with a mixture of pOGL1 (Table 7).

**TABLE 7**

| **Vaccine** | **Env-specific IFN-γ-secreting CD8⁺ T cells per 10⁶ splenocytes** |
|---|---|
| pcDNA3.1_{ZEO} | <50 |
| pOGL1-A1 | 3250 ± 354 |
| pOGL1 | 850±71 |

To further evaluate the potency of the CD8⁺ T cell response, the cytotoxic effector function of the gp120-specific CD8⁺ T cells was assessed using a short-term ⁵¹Cr-release CTL assay. The results of CTL assays corroborated with the results of the ELISPOT assays and showed that mice vaccinated with pOGL1-A1 developed a stronger CTL response than mice vaccinated with pOGL1.

### Example 9

The use of cholera toxin as an adjuvant enables the induction of long-lived humoral responses (Unpublished observation). To determine if this property was preserved in the CED vaccine, pOGL1-A1, the humoral response to gp120 was followed for 40 weeks after vaccination. Thus, a group of BALB/c mice was vaccinated intramuscularly with 100 µg-doses of endotoxin-free pOGL1-A1 DNA on weeks 0, 2, and 10. Another group of mice was vaccinated three times on weeks 0, 2, and 10 with a mixture of 100 µg pOGL1 and 100 µg pRc/CMV-*ctxA1* (expresses the A1 subunit of cholera toxin). A negative control group of 3 BALB/c mice received three 100 µg-doses of the expression vector pcDNA3.1 DNA using the same protocol.

Sera were collected before and at 1 month intervals after the first vaccination and used to measure the serum IgG response against HTV-1_{MN} gp120 by ELISA.

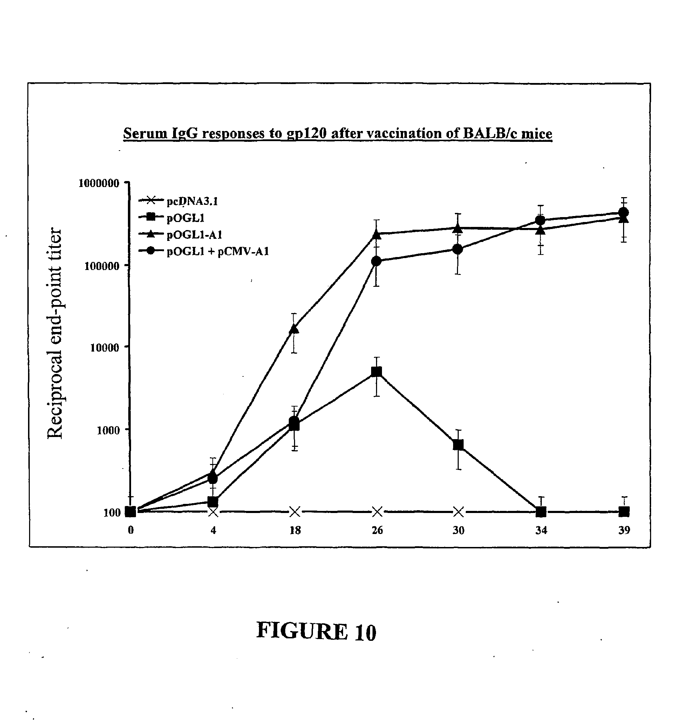

In agreement with earlier results, mice vaccinated with the dicistronic DNA vaccine pOGL1-A1 and the mixture of pOGL1 and pRc/CMV-*ctxA1* developed strong serum IgG responses against gp120 that were substantially stronger than the anti-gp120 serum IgG response that arose in pOGL1-vaccinated mice. (Figure 10). In addition, the response induced by pOGL1 waned 26 weeks after vaccination, whereas the gp120-specific serum IgG responses elicited by pOGL1-A1 and the mixture of pOGL1 and pRc/CMV-*ctxA1* remained elevated for the duration of the 40-week monitoring period. Thus, expression of a vaccine adjuvant at the cite of vaccination with a DNA vaccine, either as part of a dicistronic DNA vaccine or as part of a mixture as outlined in the example, results in the induction of long-lived humoral responses against the vaccine antigen. These data clearly demonstrate that expression of both a vaccine antigen and an adjuvant at the site of vaccination preserves the immune-stimulating properties of the adjuvant.

The contrasting effectiveness of CtxA1 (e.g., The adjuvant) in a DNA vaccine mixture (e.g., pOGL1 + pRc/CMV-A1) compared to CtxA1 co-expressed on a single plasmid with the antigen (e.g., pOGL1-A1) demonstrates the principle that DNA vaccines that co-express an antigen and an adjuvant are more effective at utilizing the immunostimulatory properties of the adjuvant than a mixture of two plasmids. The data show that co-expression of an antigen (e.g., gp120) and an adjuvant (e.g., CtxA1) effectively (and synergistically) harnesses the activity of the adjuvant. Therefore, DNA vaccines that co-express an antigen and an adjuvant are useful for the induction of strong humoral and cell-mediated immune responses against antigens.

### Example 10

### Construction of a co-expression DNA vaccine encoding an autoimmune antigen and an immunoregulatory protein

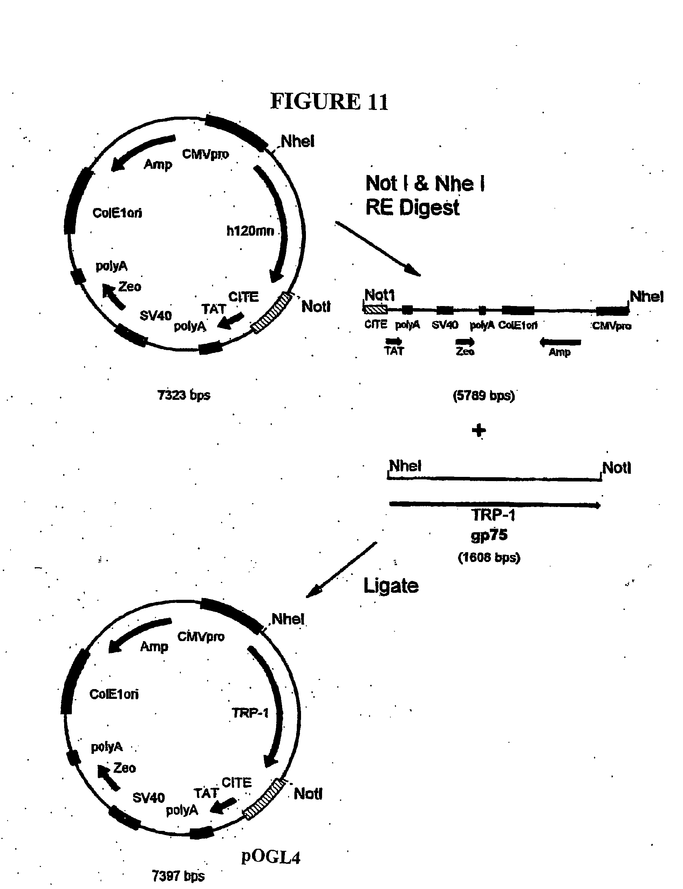

A co-expression DNA vaccine comprised of sequences encoding human Tyrosinase-Related Protein 1 (trip-1, Acc. # X51420) and the Human Immunodeficiency Virus protein Tat was constructed as follows. DNA expressing TRP-1 was cloned into the plasmid pOGL1-wT, by substituting the *hgp*120-encoding DNA (Figure 11). *Escherichia coli* containing the plasmid pHTalpha2, which expresses TRP-1, was obtained from ATCC (Manassas, VA; #65118); pHTalpha2 DNA was purified using the Qiagen Maxi Prep protocol (Valencia, CA; Cat. # 12262). PCR was then conducted to amplify the TRP-1 encoding sequence on pHTalpha2. The restriction endonuclease sites NheI(5') and NotI(3') were incorporated into the PCR-generated product by primer extension. The resulting PCR product was digested with the aforementioned enzymes (Purchased from New England Biolabs, Beverly, MA; Cat. #131S and #189S) to yield a 1608 bp fragment with *Nhe*I and *Not*I compatible ends. Plasmid pOGL1-wT was also digested with NheI and NotI, resulting in a 5789 bp product and a 1543 bp product (comprised of the plasmid backbone pcDNA3.1_{ZEO} as well as the IRES and *htat*). The TRP-1-encoding 1608 bp fragment from the above described PCR and the 5789 bp fragment were purified by gel extraction (Qiagen; Cat. # 28706). These fragments were annealed together using DNA T4 Ligase according to the manufacturer's directions (New England Biolabs; Cat. #202CS).

The *E. Coli* strain Stable2, prepared as chemically competent cells (Life Technologies, Gaithersburg MD, Cat # 10268-019), was then transformed with the 7397 bp annealed product and positive clones were selected using TSA containing Ampicillin. Isolated colonies were selected, numbered, and allowed to further incubate (30°C, 20 hrs) and plasmids were purified from these putative clones using the Qiagen Mini-Prep system (Cat # 12123). The presence of plasmids that display the appropriate restriction enzyme pattern were screened with the *Nhe*I and *Not*I restriction enzymes. Positive clones were then selected and stored at -80°C in TSB containing 30% glycerol. One of these clones, herein referred to as T212 was sent to Aldeveron (Fargo, ND) for the production of purified, endotoxin-free pOGL4 plasmid DNA vaccine. The sequence of the novel vaccine was then verified by the University of Maryland's Biopolymer Lab (Baltimore, MD).

### Example 11

### Construction of a co-expression DNA vaccine encoding an antoimmune antigen and T cell growth factor-beta

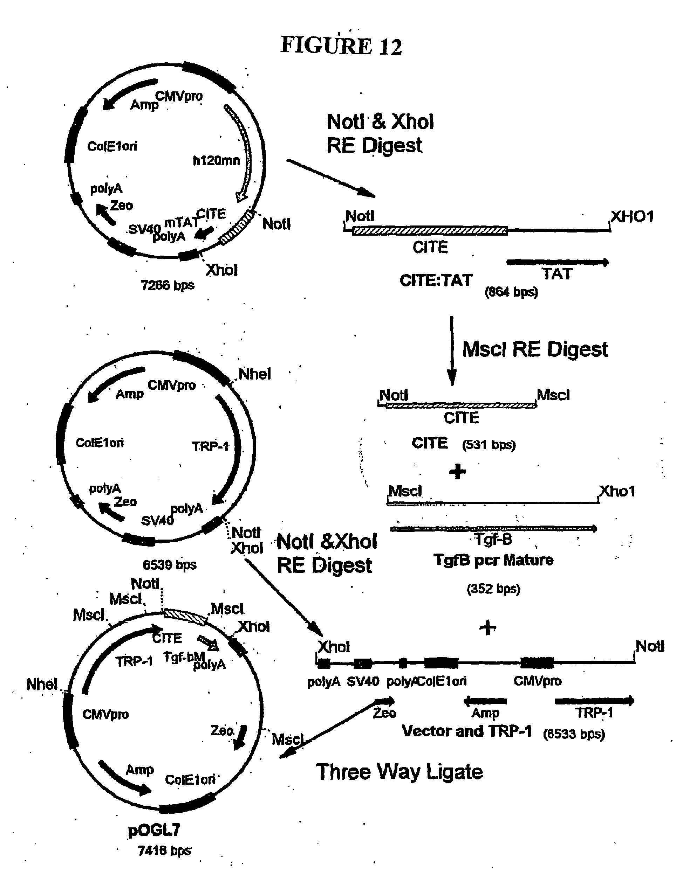

To construct a DNA vaccine comprised of sequences encoding TRP-1 and Transforming Growth Factor-β (TGF-β, Acc. # V00083), three separate DNA fragments were generated and annealed (Figure 12). The DNA fragment encoding TRP-1 is obtained by cloning a PCR generated TRP-1 fragment and inserting it into the well-known expression vector pcDNA3.1. To this end, plasmid pcDNA3.1 DNA is digested with the restriction enzymes NheI and NotI (as describe in example 6 above), to yield a 4931 bp fragment. This fragment is purified by fractionating the DNA by agarose gel electrophoresis; the 4931 by pair fragment is isolated using the Qiagen agarose purification kit. PCR is performed on pHTalpha2, following the identical protocol as used in example 6 above. This latter PCR generated product is also digested with NotI and NheI and the 1608 bp fragment is purified using the Qiagen agarose gel extraction kit. The purified 4931 bp pcDNA3.1 and 1608 bp TRP-1 fragment are subsequently annealed with T4 DNA ligase as before. *E*.*coli* strain Stable2 is transformed with the resultant 6539 product and Ampicillin resistant clones are screened by the same method as above. An isolate containing the desired recombinant plasmid, referred to here in as pOGL7, is then used as a source of large-scale plasmid DNA production that is obtained commercially from Aldeveron, LLC (Fargo, North Dakato)

This plasmid is then cleaved with the restriction enzymes NotI and XhoI (NEB, Cat. #146S) to generate the DNA fragment encoding pcDNA linked to TRP-1 and the 6533 bp fragment is purified through agarose gel extraction as above.

A second DNA fragment comprised of the sequence encoding the CITE region of the VEE virus which provides an IRES sequence and is useful for co-expression of two proteins from a single promoter. Thus, plasmid pcDNA-hm120:CITE:Tat is digested with the restriction enzyme MscI (NEB; Cat# 534S) and NotI yielding four fragments: 1467, 531, 1535, and 3790 bp in size; the 531 bp fragment contains the CITE region and is purified using the Qiagen agarose gel extraction procedure cited above. The final DNA fragment is a 370 bp DNA sequence encoding TGF-β and is obtained through PCR amplification as follows: An *E. Coli* HB101 derivative containing plasmid phTGFB-2, which encodes the entire human TGF-β product, was obtained from ATCC (Manassas VA, ATCC No. 59954). Plasmid pHTGFB-2 DNA was purified using the Qiagen MaxiPrep Protocol. The pHTGFB-2 plasmid is then used as a source and PCR is then conducted to amplify only the mature TGF-B encoding sequence. Through primer extension PCR, *Msc*I and *Xho*I restriction enzyme sites are incorporated into the PCR-generated product; the resultant 370bp fragment is cleaved with these enzymes and purified by gel extraction as described above.

Finally, the 6533 bp DNA fragment composed of pcDNA3.1::TRP-1, the 531 bp DNA. fragment encoding CITE, and the 370 bp DNA fragment encoding TGF-β are annealed together using T4 DNA ligase as above. The resulting 7434 bp recombinant plasmid is transformed into Stable2 bacteria. Ampicillin-resistant clones are then screened as above by restriction enzyme analysis and a clone containing the appropriate plasmid that displays the predicted digestion pattern by agarose gel electrophoresis is selected and stored at -80 C in TSB containing 30% (v/v) glycerol. A large scale preparation of this plasmid, here in referred to as pcDNA:TRP-1::CITE::TGF-β, for vaccination of mice is purchased from Adeveron; automated DNA sequencing of the TRP-1::CITE::TGF-β fragment is conducted to verify the structural integrity of this DNA prior to vaccination of the mice.

### Example 12

### Construction of a co-expression DNA vaccine encoding a vaccine antigen and an immunostimulatory adjuvant

Novel DNA vaccines were constructed that co-expresses an antigen (e.g., gp120) and an adjuvant, e.g., enzymatically active domains of *E*. *coli* heat labile enterotoxin (LT), pertussis toxin (PT) from *B. pertussis* and the adenylate cyclase toxin (cya) from *B. pertussi.* LT is highly homologous to cholera toxin (CT) and like CT, leads to the upregulation of intracellular cAMP levels through the ADP-ribosylation of Gsα which in turn constitutively activates adenylate cyclase. PT increases intracellular cAMP levels through the ADP-ribosylation of Gi which in turn prevents the inhibition of adenylate cyclase. Adenylate cyclase toxin increases intracellular cAMP by transferring a functional adenylate cyclase enzyme into the cytoplasm of target cells.

All procedures used in the construction of pOGL1-A1 hereinabove, including restriction endonuclease digestions, agarose gel electrophoresis, DNA purification, DNA ligation and PCR were generally followed.

The nucleotide sequence of the A subunit of LT was obtained from Pub Med (Accession # M57244 SEQ ID. NO: 5). The A1 subunit starts at position 1 and the coding sequence extends through position 582. The A2 subunit starts at position 583 and the coding sequence extends through position 723. The following PCR primers were made to clone the LTA1 subunit into the eukaryotic expression plasmid pRc/CMV. The forward primer 5' CGC GCG AAGCTT ATG GGC GAC AGA TTA TAC CGT GCT GAC TCT (SEQ ID NO: 6) and reverse primer 5' CGC GCG GCGGCCGC TTA TTA GAT TGT TCT TGA TGA ATT TCC (SEQ ID NO: 7). The forward primer has a Hind III restriction enzyme site and the reverse primer has a Not I restriction enzyme site.

The plasmid used for the PCR template was pCVD 403 and provided by the Center for Vaccine Development, University of Maryland and which contained the gene encoding LT. The same method described for cloning CTA1 into pRc/CMV was used to clone LTA1 into pRc/CMV.

The nucleotide sequence PT was obtained from Pub Med (Accession # E01352, SEQ ID NO: 8). The S1 subunit starts at position 609 and the coding sequence extends through position 1313. The following PCR primers were made to clone the PTS1 subunit into the eukaryotic expression plasmid pRC/CMV. The primers used included the forward primer 5' CGC GCG AAGCTT ATG GAC GAT CCT CCC GCC ACC GTA TAC CGC (SEQ ID NO: 9) and the reverse primer CGC GCG GCGGCCGCC TTA TTA GAA CGA ATA CGC GAT GCT TTC GTA (SEQ ID NO: 10). The forward primer has a Hind III restriction enzyme site and the reverse primer has a Not I restriction enzyme site.

The plasmid used for the PCR template was pBLUE/S1wt was provided by the Center for Vaccine Development, University of Maryland and which contained the gene encoding PTS1. The same method described for cloning CTA1 into pRC/CMV was used to clone PTS1 into pRC/CMV.

The nucleotide sequence the *B. pertussis* cya gene was obtained from Pub Med (Accession # Y00545, SEQ ID NO: 11). cyaA contains the enzymatically active adenylate cyclase enzyme of adenylate cyclase toxin and the enzymatic activity is confined to the first 400 amino acids (Hanski, TIBS 1989). The coding sequence for the first 400 amino acids of cyaA starts at position 981 and extends through position 2181. The following PCR primers were made to clone the first 400 amino acids of cyaA into the eukaryotic expression plasmid pRC/CMV, forward primer CGC GCG AAGCTT ATG CAG CAA TCG CAT CAG GCT GGT TAC (SEQ ID NO: 12) and reverse primer CGC GCG GCGGCCGC TTA TTA CTG GCG TTC CAC TGC GCC CAG (SEQ ID NO: 13). The forward primer has a Hind III restriction enzyme site and the reverse primer has a Not I restriction enzyme site.

The plasmid used for the PCR template was pBLUE/cya/cla was provided by the Center for Vaccine Development, University of Maryland) which contained the gene encoding PTS1. The same method described for cloning CTA1 into pRC/CMV was used to clone the first 400 amino acids of cyaA into pRC/CMV.

DNA Vaccine Adjuvant Studies: After the DNA vaccine constructs have been cloned, they will be grown in a large scale and purified. The constructs will also be tested for endotoxin contamination. An example of a DNA vaccine adjuvant study will be outlined below.

The animal model will be BALB/C or C57BL6 mice. Each experimental and control group will be made up of 5 mice. The following control groups will be used in each study.
1. A negative control group that will receive saline during immunizations.
2. An antigen alone group that will be immunized with the plasmid expressing the antigen of interest (OVA, GP-120, etc.).
3. An adjuvant alone group that will be immunized with the plasmid expressing the putative adjuvant (CTA1, LTA1, PTS1 etc.), There will be a separate group for each adjuvant being tested in the study.

The experimental groups used in the study will be co-immunized with the plasmid expressing the antigen and with the plasmid expressing the putative adjuvant. There will be a separate experimental group for each adjutant being tested.

To begin the study each animal from each group will receive the first intra-muscular immunization. The control mice receiving one plasmid alone will be immunized with 100 µg of the respective plasmid. The experimental mice will be co-immunized with 100 µg of the antigen expressing plasmid mixed with 100 µg of the adjuvant expressing plasmid The volume of the injection will be held constant between groups and the negative control group will receive an equal volume of saline. Four teen days later the above immunization will be repeated. At 14 day intervals sera will be collected from individual mice and will be screened for anti-antigen Ig subclasses by ELISA. After the final collection of sera the mice will be sacrificed and the spleens will be harvested. The spleens will be screened for antigen specific T cells.

## Claims

1. A dicistronic DNA vaccine including:
a plasmid comprising:
an HIV antigen-encoding region encoding an antigen component, wherein the antigen component is gp120 or hgp 120; and
a biologically-active component-encoding region encoding an immunoregulatory peptide;
wherein the immunoregulatory peptide is the A1 domain of the A subunit of cholera toxin or the A subunit of E. *coli* heat labile enterotoxin.

2. The DNA vaccine of claim 1, wherein the A1 domain of the A subunit of cholera toxin is a fragment thereof which retains the biological activity of the native domain.

3. The DNA vaccine of claim 1, wherein the antigen-encoding region is operatively linked to a promoter, which drives transcription in one or more eukaryotic cell types,

4. The DNA vaccine of claim 1, wherein the biologically-active component-encoding region is operatively linked to a promoter, which drives transcription in one or more eukaryotic cell types.

5. The DNA vaccine of claim 1, wherein the antigen-encoding region is separated from the biologically-active component-encoding region by a coding region comprising a nucleotide segment encoding an internal ribosome entry sequence.

6. The DNA vaccine of claim 5, wherein the internal ribosome entry sequence is a cap-independent translational enhancer.

7. A single expression cassette including a nucleotide sequence comprising:
an HIV antigen-encoding region encoding an antigen component, wherein the antigen encoding component is gp120 or hgp120; and
a biologically-active component-encoding region encoding a component
selected from the group consisting of immunoregulatory peptides wherein the immunoregulatory peptide is the A1 domain of the A subunit of cholera toxin or the A subunit of *E*. *coli* heat labile enterotoxin.

8. The expression cassette of claim 7, wherein the antigen-encoding region is operatively linked to a promoter, which drives transcription in one or more eukaryotic cell types.

9. The expression cassette of claim 7, wherein the biologically-active component encoding region is operatively linked to a promoter, which drives transcription in one or more eukaryotic cell types.

10. The expression cassette of claim 7, wherein the antigen-encoding region is separated from the biologically-active component encoding region by a coding region comprising a nucleotide segment encoding an internal ribosome entry sequence.

11. The expression cassette of claim 10, wherein the internal ribosome entry sequence is a cap-independent translational enhancer.

12. The expression cassette according to claim 7, further comprising a plasmid backbone.

13. The expression cassette according to claim 12, wherein the plasmid backbone is selected from the group consisting of: pBR322, pUC19, pcDNA3.1ZEO, pIZc/CMV, pXTl, pSG5, pPUR, pMAM, pDual, pG51uc, pACT, pBIND, pCI-Neo, pCMV-BD, pIRES-P, pRL-CMV.

14. The expression cassette according to claim 7, comprising SEQ ID NO: 2 and SEQ ID NO: 21.

15. An expression vector for use in increasing an immune response to an HIV virus in a subject, wherein the expression vector comprises an HIV antigen-encoding region encoding an antigen component, wherein the antigen-encoding component is gp120 or hgp120; and a biologically-active component-encoding region encoding an immunoregulatory peptide, wherein the immunoregulatory peptide is the A1 domain of the A subunit of cholera toxin or the A subunit of *E. coli* heat labile enterotoxin, which retains the biological activity of the native domain.

## Patentansprüche

1. Dicistronischer DNA-Impfstoff, enthaltend:
ein Plasmid, umfassend:
eine für ein HIV-Antigen kodierende Region, die für eine Antigenkomponente kodiert, wobei die Antigenkomponente pg120 oder hgp120 ist; und
eine für eine biologisch aktive Komponente kodierende Region, die für ein immunregulatorisches Peptid kodiert;
wobei das immunregulatorische Peptid die A1-Domäne der A-Untereinheit des Choleratoxins oder die A-Untereinheit des wärmeinstabilen Enterotoxins von E. coli ist.

2. DNA-Impfstoff nach Anspruch 1, wobei die A1-Domäne der A-Untereinheit des Choleratoxins ein Fragment davon ist, das die biologische Aktivität der nativen Domäne bewahrt.

3. DNA-Impfstoff nach Anspruch 1, wobei die für das Antigen kodierende Region in Wirkverbindung mit einem Promotor steht, der die Transkription in einer oder mehreren eukaryontischen Zelltypen steuert.

4. DNA-Impfstoff nach Anspruch 1, wobei die für die biologisch aktive Komponente kodierende Region in Wirkverbindung mit einem Promotor steht, der die Transkription in einer oder mehreren eukaryontischen Zelltypen steuert.

5. DNA-Impfstoff nach Anspruch 1, wobei die für das Antigen kodierende Region von der für die biologisch aktive Komponente kodierenden Region durch eine kodierende Region getrennt ist, die ein Nukleotidsegment umfasst, das für eine interne Ribosomeneintrittssequenz kodiert.

6. DNA-Impfstoff nach Anspruch 5, wobei die interne Ribosomeneintrittssequenz ein cap-unabhängiger translationaler Enhancer ist.

7. Einzelne Expressionskassette mit einer Nukleotidsequenz, umfassend:
eine für ein HIV-Antigen kodierende Region, die für eine Antigenkomponente kodiert, wobei die für das Antigen kodierende Komponente pg120 oder hgp120 ist; und
eine für eine biologisch aktive Komponente kodierende Region, die für eine Komponente kodiert, die aus der Gruppe bestehend aus
immunregulatorischen Peptiden ausgewählt ist,
wobei das immunregulatorische Peptid die A1-Domäne der A-Untereinheit des Choleratoxins oder die A-Untereinheit des wärmeinstabilen Enterotoxins von E. coli ist.

8. Expressionskassette nach Anspruch 7, wobei die für das Antigen kodierende Region in Wirkverbindung mit einem Promotor steht, der die Transkription in einer oder mehreren eukaryontischen Zelltypen steuert.

9. Expressionskassette nach Anspruch 7, wobei die für die biologisch aktive Komponente kodierende Region in Wirkverbindung mit einem Promotor steht, der die Transkription in einer oder mehreren eukaryontischen Zelltypen steuert.

10. Expressionskassette nach Anspruch 7, wobei die für das Antigen kodierende Region von der für die biologisch aktive Komponente kodierenden Region durch eine kodierende Region getrennt ist, die ein Nukleotidsegment umfasst, das für eine interne Ribosomeneintrittssequenz kodiert.

11. Expressionskassette nach Anspruch 10, wobei die interne Ribosomeneintrittssequenz ein cap-unabhängiger translationaler Enhancer ist.

12. Expressionskassette nach Anspruch 7, weiter ein Plasmidgerüst umfassend.

13. Expressionskassette nach Anspruch 12, wobei das Plasmidgerüst aus der Gruppe bestehend aus: pBR322, pUC19, pcDNA3.1ZEO, pRc/CMV, pXT1, pSG5, pPUR, pMAM, pDual, pG51uc, pACT, pBIND, pCl-Neo, pCMV-BD, pIRES-P, pRL-CMV ausgewählt ist.

14. Expressionskassette nach Anspruch 7, umfassend SEQ ID NO: 2 und SEQ ID NO: 21.

15. Expressionsvektor zur Verwendung zum Steigern einer Immunreaktion auf ein HIV-Virus bei einem Patienten, wobei der Expressionsvektor eine für ein HIV-Antigen kodierende Region umfasst, die für eine Antigenkomponente kodiert, wobei die für das Antigen kodierende Komponente gp120 oder hgp120 ist; sowie eine für eine biologisch aktive Komponente kodierende Region, die für ein immunregulatorisches Peptid kodiert, wobei das immunregulatorische Peptid die Al-Domäne der A-Untereinheit des Choleratoxins oder die A-Untereinheit des wärmeinstabilen Enterotoxins von E. coli ist, die die biologische Aktivität der nativen Domäne bewahrt.

## Revendications

1. Vaccin à base d'ADN dicistronique incluant :
- un plasmide comprenant :
- une région codeuse d'antigène de VIH codant un composant d'antigène, pour lequel le composant d'antigène est un gp120 ou un hgp120 ; et
- une région biologiquement active codeuse de composant codant un peptide immunorégulateur ;
- pour lequel le peptide immunorégulateur est le domaine A1 de la sous-unité A d'une toxine de choléra ou de la sous-unité A d'une entérotoxine thermolabile de *E. coli.*

2. Vaccin à base d'ADN selon la revendication 1, pour lequel le domaine A1 de la sous-unité A d'une toxine de choléra est un fragment de celui-ci qui conserve l'activité biologique du domaine à l'état natif.

3. Vaccin à base d'ADN selon la revendication 1, pour lequel la région codeuse d'antigène est liée de façon fonctionnelle à un promoteur, qui entraîne la transcription dans un ou plusieurs types de cellules eucaryotiques.

4. Vaccin à base d'ADN selon la revendication 1, pour lequel la région biologiquement active codeuse de composant est liée de façon fonctionnelle à un promoteur, qui entraîne la transcription dans un ou plusieurs types de cellules eucaryotiques.

5. Vaccin à base d'ADN selon la revendication 1, pour lequel la région codeuse d'antigène est séparée de la région biologiquement active codeuse de composant par une région codeuse comprenant un segment de nucléotide codant une séquence d'entrée de ribosome interne.

6. Vaccin à base d'ADN selon la revendication 5, pour lequel la séquence d'entrée de ribosome interne est un amplificateur translationel indépendant de la coiffe.

7. Cassette d'expression unique incluant une séquence de nucléotide, comprenant :
- une région codeuse d'antigène de VIH codant un composant d'antigène, pour laquelle le composant codeur d'antigène est un gp120 ou un hgp120 ; et
- une région biologiquement active codeuse de composant codant un composant sélectionné parmi le groupe constitué de peptides immunorégulateurs ;
- pour laquelle le peptide immunorégulateur est le domaine A1 de la sous-unité A d'une toxine de choléra ou de la sous-unité A d'une entérotoxine thermolabile de *E. coli.*

8. Cassette d'expression selon la revendication 7, pour laquelle la région codeuse d'antigène est liée de façon fonctionnelle à un promoteur, qui entraîne la transcription dans un ou plusieurs types de cellules eucaryotiques.

9. Cassette d'expression selon la revendication 7, pour laquelle la région biologiquement active codeuse de composant est liée de façon fonctionnelle à un promoteur, qui entraîne la transcription dans un ou plusieurs types de cellules eucaryotiques.

10. Cassette d'expression selon la revendication 7, pour laquelle la région codeuse d'antigène est séparée de la région biologiquement active codeuse de composant par une région codeuse comprenant un segment de nucléotide codant une séquence d'entrée de ribosome interne.

11. Cassette d'expression selon la revendication 10, pour laquelle la séquence d'entrée de ribosome interne est un amplificateur translationel indépendant de la coiffe.

12. Cassette d'expression selon la revendication 7, comprenant, en outre, une épine dorsale de plasmide.

13. Cassette d'expression selon la revendication 12, pour laquelle l'épine dorsale de plasmide est sélectionnée parmi le groupe constitué par : pBR322, pUC19, pcDNA3.IZEO, pRc/CMV, pXT1, pSG5, pPUR, pMAM, pDual, pG51uc, pACT, pBIND, pCI-Neo, pCMV-BD, pIRES-P et pRL-CMV.

14. Cassette d'expression selon la revendication 7, comprenant une SEQ ID NO : 2 et une SEQ ID NO : 21.

15. Vecteur d'expression destiné à être utilisé pour accroître une réponse immunitaire à un virus de VIH chez un sujet, pour lequel le vecteur d'expression comprend une région codeuse d'antigène de VIH codant un composant d'antigène, pour lequel le composant codeur d'antigène est un gp120 ou un hgp120 ; et une région biologiquement active codeuse de composant codant un peptide immunorégulateur, pour lequel le peptide immunorégulateur est le domaine A1 de la sous-unité A d'une toxine de choléra ou de la sous-unité A d'une entérotoxine thermolabile de *E*. *coli,* qui conserve l'activité biologique du domaine à l'état natif.


### SEQUENCE LISTING

<110> HONE, DAVID M.
<120> GENETICALLY ENGINEERED CO-EXPRESSION DNA VACCINES, C ONSTRUCTION METHODS AND USES THEREOF
<130> 4115-128
<160> 23
<170> PatentIn version 3.1
<210> 1
   <211> 723
   <212> DNA
   <213> Vibrio cholerae
<400> 1
<210> 2
   <211> 582
   <212> DNA
   <213> Vibrio cholerae
<400> 2
<210> 3
   <211> 27
   <212> DNA
   <213> Artificial Sequence
<220>
   <223> Primer <400> 3
<210> 4
   <211> 29
   <212> DNA
   <213> Artificial Sequence
<400> 4
   ggggcggccg cttacgatga tcttggagc 29
<210> 5
   <211> 723
   <212> DNA
   <213> Escherichia coli
<400> 5
<210> 6
   <211> 42
   <212> DNA
   <213> Artificial Sequence
<220>
   <223> primer
<400> 6
   cgcgcgaagc ttatgggcga cagattatac cgtgctgact ct 42
<210> 7
   <211> 41
   <212> DNA
   <213> Artificial Sequence
<220>
   <223> primer
<400> 7
   cgcgcggcgg ccgcttatta gattgttctt gatgaatttc c
   41
<210> 8
   <211> 4696
   <212> DNA
   <213> Bordetella pertussis
<400> 8
<210> 9
   <211> 42
   <212> DNA
   <213> Artificial Sequence
<220>
   <223> primer
<400> 9
   cgcgcgaagc ttatggacga tcctcccgcc accgtatacc gc 42
<210> 10
   <211> 44
   <212> DNA
   <213> Artificial Sequence
<220>
   <223> primer
<400> 10
   cgcgcggcgg ccgcttatta gaacgaatac gcgatgcttt cgta 44
<210> 11
   <211> 6442
   <212> DNA
   <213> Bordetella pertussis
<400> 11
<210> 12
   <211> 39
   <212> DNA
   <213> Artificial Sequence
<220>
   <223> primer
<400> 12
   cgcgcgaagc ttatgcagca atcgcatcag gctggttac 39
<210> 13
   <211> 41
   <212> DNA
   <213> Artificial Sequence
<220>
   <223> primer
<400> 13
   cgcgcggcgg ccgcttatta ctggcgttcc actgcgccca g 41
<210> 14
   <211> 39
   <212> DNA
   <213> Artificial Sequence
<220>
   <223> primer
<400> 14
   ggggggggat ccatgcccat ggggtctctg caaccgctg
   39
<210> 15
   <211> 44
   <212> DNA
   <213> Artificial Sequence
<220>
   <223> primer
<400> 15
   gggggcggcc gcttattagg cgcgcttctc gcgctgcacc acgc
   44
<210> 16
   <211> 71
   <212> DNA
   <213> Artificial Sequence
<220>
   <223> primer
<400> 16
<210> 17
   <211> 42
   <212> DNA
   <213> Artificial Sequence
<220>
   <223> primer
<400> 17
   gccaaataca tggccatatt atcatcgtgt ttttcaaagg aa
   42
<210> 18
   <211> 319
   <212> DNA
   <213> Human immunodeficiency virus type 1
<400> 18
<210> 19
   <211> 46
   <212> DNA
   <213> Artificial Sequence
<220>
   <223> primer
<400> 19
   gccaaataca tggccattga gcccgtggac cctcgcctgg agccct 46
<210> 20
   <211> 42
   <212> DNA
   <213> Artificial Sequence
<220>
   <223> primer
<400> 20
   ataagaatct cgagcagctg gaattcgcgg ccggctgatc ag 42
<210> 21
   <211> 1518
   <212> DNA
   <213> Human immunodeficiency virus
<400> 21
<210> 22
   <211> 1518
   <212> DNA
   <213> Human immunodeficiency virus type 1
<400> 22
<210> 23
   <211> 213
   <212> PRT
   <213> Human immunodeficiency virus type 1
<220>
   <221> MISC_FEATURE
   <223> n can be any amino acic
<220>
   <221> MISC_FEATURE
   <222> (1)..(213)
   <223> n can be any amino acic
<400> 23
   Xaa Xaa Xaa Cys Glu Asp Met Glu Lys Glu Gly Lys Ile Ser Lys
   Ile
   1 5 10 15
   Gly Pro Glu Asn Pro Tyr Asn Thr Pro Val Phe Ala Ile Lys Lys
   Lys
   20 25 30
   Asp Ser Thr Lys Trp Arg Lys Leu Val Asp Phe Arg Glu Leu Asn
   Lys
   35 40 45
   Arg Thr Gln Asp Phe Trp Glu Val Gln Leu Gly Ile Pro His Pro
   Ala
   50 55 60
   Gly Leu Lys Lys Lys Lys Ser Val Thr Val Leu Asp Val Gly Asp
   Ala
   65 70 75
   80
   Tyr Phe Ser Val Pro Leu Asp Glu Asn Phe Arg Lys Tyr Thr Ala
   Phe
   85 90 95
   Thr Ile Pro Ser Ile Asn Asn Glu Thr Pro Gly Ile Arg Tyr Gln
   Tyr
   100 105 110
   Asn Val Leu Pro Gln Gly Trp Lys Gly Ser Pro Ser Ile Phe Gln
   Gly
           115                 120                 125
   Ser Met Thr Lys Ile Leu Glu Pro Phe Arg Ala Arg Asn Pro Glu
   Ile
      130                 135                 140
   Val Ile Tyr Gln Tyr Met Asp Asp Leu Tyr Val Gly Ser Asp Leu
    Glu
   145                 150                 155
    160
   Ile Gly Gln His Arg Ala Lys Ile Glu Glu Leu Arg Glu His Leu
   Leu
                  165                 170                 175
   Lys Trp Gly Phe Thr Thr Pro Asp Lys Lys His Gln Lys Glu Pro
   Pro
              180                 185                 190
   Phe Leu Trp Met Gly Tyr Glu Leu His Pro Asp Lys Trp Thr Val
   Gln
          195                 200                 205
   Pro Ile Glu Leu Pro
       210